# EUROPEAN PATENT SPECIFICATION

(11) **EP 4 647 109 B1**
(45) Date of publication and mention of the grant of the patent: **28.10.2026**
(21) Application number: 24853038.8
(22) Date of filing: 07.08.2024
(51) Int. Cl.: A61M 60/804, A61M 60/13, A61M 60/237, A61M 60/414, A61M 60/419, A61M 60/585, A61M 60/824, A61M 60/825, A61M 60/829

(54) **CATHETER PUMP**
KATHETERPUMPE
POMPE DE CATHÉTER

(30) Priority: 19.03.2024 CN 202410309425
(43) Date of publication of application: 12.11.2025
(73) Proprietor: Shanghai EnginPrime Medtec CO., LTD., Shanghai 200032 (CN)
(72) Inventor: ZHENG, Xiaojuan, Chengdu, Sichuan 610095 (CN); ZHENG, Tao, Chengdu, Sichuan 610095 (CN)
(74) Representative: M. Zardi & Co S.A.
(86) International application number: PCT/CN2024/110243
(87) International publication number: WO 2025/194661

(56) References cited:
- WO-A1-2009/157840
- WO-A1-2021/127503
- WO-A1-2021/152013
- WO-A1-2022/189585
- WO-A1-2023/091452
- WO-A1-2024/037119
- WO-A1-2024/037203
- WO-A2-2019/138350
- CN-A- 112 891 732
- CN-A- 114 225 214
- CN-A- 115 999 045
- CN-A- 116 328 176
- CN-A- 116 474 256
- CN-A- 116 726 377
- CN-A- 116 726 377
- CN-A- 116 887 881
- US-A1- 2023 043 385
- US-A1- 2023 256 230

## Description

### TECHNICAL FIELD

The present disclosure relates to the technical field of catheter pumps.

### BACKGROUND

Percutaneous coronary intervention (PCI) is a commonly used and effective method to treat coronary heart disease. Compared with heart bypass surgery, the PCI surgery has a lower risk, less trauma, lower surgical difficulty and faster postoperative recovery. In addition, the PCI surgery is also suitable for the rescue of acute myocardial infarction. During the surgery, a blood pumping device can provide patients with more stable blood circulation support, improve the perfusion of coronary artery and distal organs while reducing the burden on the heart, which is conducive to the stability of physical signs of patients during the surgery and postoperative rehabilitation, thus promoting the recovery of hearts of patients.

A percutaneous blood pumping device is a miniature blood pumping device, and the blood pumping performance of which is determined by the operation mode rather than dependent on the physical state of patients. The device belongs to an active blood circulation support instrument. In order to provide sufficient blood support, a power output component of the blood pumping device needs to provide a high enough rotation speed, but the higher rotation speed results in the worse hemolytic performance of the tissue. In addition, the study shows that on the condition that the delivery size of the miniature blood pumping device at the position of the interventional puncture point is small enough, vascular complications can be reduced and the safety and the effectiveness of the miniature blood pumping device can be improved.

Based on the above clinical reality, the blood pumping device needs to meet the requirements of minimally invasive operation, a low rotation speed, low hemolysis and a large blood pumping flow rate. A compressible blood pumping catheter is known. Prior to entering the human body, the blood pumping catheter is compressed into a compressed state by an external force to achieve minimally invasive intervention. Through the percutaneous surgery, the blood pumping catheter is entered into the designated position of the human body in a compressed state, and the blood pumping catheter is restored to an expanded state, so that the actual blood pumping channel is enlarged, thus meeting the requirements of a low rotation speed, low hemolysis and a large blood pumping flow rate. The core component of the blood pumping catheter is the compressible impeller, but there are many problems in the existing compressible blood pumping catheter.

In the prior art, due to the poor binding force between the rotating shaft and the impeller, the impeller moves relatively with respect to the impeller rotating shaft, so that a relative rotation takes place between the impeller and the impeller rotating shaft, and the working efficiency of the impeller is reduced. The forward and backward movement of the impeller causes the impeller to scrape with the housing, increasing the risk of mechanical hemolysis.

CN 116 726 377 A is an example of a catheter pump according to the prior art.

### SUMMARY

A catheter pump is disclosed. A problem of a poor binding force between a rotating shaft and an impeller in the prior art is solved through the integral injection molding of the rotating shaft and the impeller.

A catheter pump according to the present invention is claimed in claim 1.

Advantageous embodiments of the catheter pump according to the present invention are claimed in claims from 2 to 30.

The catheter pump as disclosed includes a functional module. The functional module includes an impeller structure, and the impeller structure includes an impeller rotating shaft and an impeller. The impeller includes a hub and several blades, the several blades are arranged on the hub. A side wall of the impeller rotating shaft is provided with a connecting part, and the hub is connected with the connecting part by integral injection molding. The connecting part is configured to limit relative movement of the impeller.

In some embodiments, the catheter pump further includes a pigtail pipe, an expandable bracket, a housing, a multi-cavity pipe and a handle module. The impeller rotating shaft is rotatably connected with the pigtail pipe, the impeller rotating shaft is connected with a mechanical power source in the handle module through a transmission shaft in the multi-cavity pipe. The expandable bracket is located at an outer side of the impeller structure, a distal end of the expandable bracket is connected with the pigtail pipe, and a proximal end of the expandable bracket is connected with the multi-cavity pipe. The housing is located at an outer side of the expandable bracket, the housing is connected with the pigtail pipe and the multi-cavity pipe, respectively, and the housing is provided with a blood inlet window and a blood outlet window.

In some embodiments, a distal end of the impeller rotating shaft is provided with a rotating shaft step, the impeller rotating shaft is rotatably connected with the pigtail pipe through a rotating structure, and a proximal end of the rotating structure is matched with the rotating shaft step to axially limit the impeller rotating shaft and restrict the impeller rotating shaft from moving to a distal end of the catheter pump.

The catheter pump as disclosed includes a functional module and a liquid path module. The functional module includes an impeller structure. The impeller structure includes an impeller rotating shaft and an impeller, and the impeller includes a hub and several blades. A rotating structure is provided between a distal end of the impeller rotating shaft and a pigtail pipe. The several blades are provided on the hub, a side wall of the impeller rotating shaft is provided with a connecting part. The hub is connected with the connecting part by integral injection molding, and the connecting part is configured to limit relative movement of the impeller. The liquid path module includes a multi-cavity pipe, and the multi-cavity pipe includes a perfusion cavity. Perfusion liquid flows into a distal end of the impeller rotating shaft from the perfusion cavity, flows out from a gap between the impeller rotating shaft and the rotating structure, and then enters a human body.

In some embodiments, the catheter pump further includes an expandable bracket, a housing and a handle module. The impeller rotating shaft is connected with a mechanical power source in the handle module through a transmission shaft in the multi-cavity pipe. The expandable bracket is located at an outer side of the impeller structure. A distal end of the expandable bracket is connected with the pigtail pipe, and a proximal end of the expandable bracket is connected with the multi-cavity pipe. The housing is located at an outer side of the expandable bracket, the housing is connected with the pigtail pipe and the multi-cavity pipe, respectively, and the housing is provided with a blood inlet window and a blood outlet window.

In some embodiments, the multi-cavity pipe is further provided with an outflow cavity. The perfusion cavity extends from one end of the multi-cavity pipe to an other end of the multi-cavity pipe. The outflow cavity extends from one end of the multi-cavity pipe to an other end of the multi-cavity pipe. A proximal end of the impeller rotating shaft is connected with a distal end of the transmission shaft. A liquid path channel is arranged in the impeller rotating shaft, the impeller rotating shaft is provided with an inlet communicated with the liquid path channel. The inlet is communicated with the perfusion cavity, the liquid path channel and the outflow cavity, respectively. Perfusion liquid flows from the perfusion cavity, flows into the liquid path channel from the proximal end of the impeller rotating shaft through the inlet, flows along the liquid path channel to the distal end of the impeller rotating shaft, flows out from the gap between the impeller rotating shaft and the rotating structure, and then enters the human body. Perfusion liquid flows out from the outflow cavity.

In some embodiments, the multi-cavity pipe includes an inner pipe and an outer pipe, the inner pipe is coaxially arranged at an inner side of the outer pipe, the perfusion cavity is arranged between the inner pipe and the outer pipe, the transmission shaft is located in the inner pipe, and the outflow cavity is provided between the transmission shaft and the inner pipe.

In some embodiments, a spring pipe is formed between the transmission shaft and the inner pipe.

In some embodiments, the proximal end of the impeller rotating shaft is connected with the distal end of the transmission shaft through a switching block, the switching block is provided with a first channel, and the first channel is communicated with the inlet, the perfusion cavity and the outflow cavity, respectively.

In some embodiments, the proximal end of the impeller rotating shaft is provided with protrusions, the inlet is formed between the adjacent protrusions, the switching block is provided with a chute, the protrusion is in sliding connection with the chute, a second channel is formed between an inner surface of the protrusion and a surface of the chute, and the second channel is communicated with the perfusion cavity and the liquid path channel, respectively.

In some embodiments, the second channel is further communicated with the first channel and the outflow cavity.

In some embodiments, a sheath cover is arranged at an outer side of a joint between the proximal end of the impeller rotating shaft and the switching block, the sheath cover is connected with the expandable bracket and the multi-cavity pipe, respectively, an opening is formed on the sheath cover, and a distal end of the perfusion cavity is communicated with a distal end of the outflow cavity through the opening.

In some embodiments, the sheath cover is provided with a sheath cover limiting step, the sheath cover limiting step is matched with the proximal end of the impeller rotating shaft and the proximal end of the switching block to axially limit the impeller rotating shaft and restrict the impeller rotating shaft from moving to the proximal end of the catheter pump.

In some embodiments, a third channel is arranged between the proximal end of the impeller rotating shaft and the sheath cover, a proximal end of the third channel is communicated with the perfusion cavity, and the distal end of the third channel is communicated with the human body.

In some embodiments, the third channel is further communicated with the first channel and the outflow cavity.

The catheter pump as disclosed includes a functional module and a handle module. The functional module includes an impeller structure, and the impeller structure includes an impeller rotating shaft and an impeller. The impeller includes a hub and several blades, and the several blades are arranged on the hub. A side wall of the impeller rotating shaft is provided with a connecting part. The hub is connected with the connecting part by integral injection molding, and the connecting part is configured to limit relative movement of the impeller. The handle module includes a transmission sealing structure. The transmission sealing structure includes an external magnetic component, an internal magnetic component, a driven shaft and a sealing cover. The internal magnetic component is arranged on the driven shaft, and the driven shaft is connected with the impeller rotating shaft through a transmission shaft. The external magnetic component is arranged at an outer side of the internal magnetic component, and the external magnetic component is connected with a mechanical power source. The sealing cover is configured to isolate the internal magnetic component from the external magnetic component. The driven shaft rotates with respect to the sealing cover and moves in an axial direction.

In some embodiments, the catheter pump further includes a pigtail pipe, an expandable bracket, a housing and a liquid path module. The impeller rotating shaft is rotatably connected with the pigtail pipe through a rotating structure. The liquid path module includes a multi-cavity pipe. The transmission shaft is located in the multi-cavity pipe. The expandable bracket is located at an outer side of the impeller structure. A distal end of the expandable bracket is connected with the pigtail pipe, and a proximal end of the expandable bracket is connected with the multi-cavity pipe. The housing is located at an outer side of the expandable bracket, and the housing is connected with the pigtail pipe and the multi-cavity pipe, respectively. The housing is provided with a blood inlet window and a blood outlet window.

In some embodiments, the handle module further includes a perfusion cavity body. A perfusion cavity of the multi-cavity pipe is communicated with a perfusion port of the perfusion cavity body. An outflow cavity of the multi-cavity pipe is communicated with an outflow port of the perfusion cavity body. A proximal end of the multi-cavity pipe is connected with the perfusion cavity body. A proximal end of the perfusion cavity is not communicated with a proximal end of the outflow cavity. The sealing cover is connected with the perfusion cavity body.

In some embodiments, the internal magnetic component is sleeved on the driven shaft and is fixedly connected with the driven shaft, the sealing cover is arranged at an outer side of the internal magnetic component, the external magnetic component is arranged at an outer side of the sealing cover, an opening end of the sealing cover is hermetically connected with the perfusion cavity body, and both the driven shaft and the internal magnetic component are capable of moving in an axial direction of the sealing cover.

In some embodiments, one end of the driven shaft protrudes from the opening end of the sealing cover, a gap is formed between the driven shaft and the perfusion cavity body, and the gap between the driven shaft and the perfusion cavity body is communicated with the outflow cavity. An other end of the driven shaft is close to a sealing end of the sealing cover. A proximal end of the impeller rotating shaft is fixedly connected with a distal end of the transmission shaft. The movement of the driven shaft is limited by the sealing end. Gaps are formed between the driven shaft and the sealing cover, and between the internal magnetic component and the sealing cover, respectively.

In some embodiments, the driven shaft has a solid structure, and one end of the driven shaft is connected with the transmission shaft.

In some embodiments, the driven shaft has a hollow structure, and the transmission shaft extends into the driven shaft.

In some embodiments, the transmission shaft is a hollow shaft, a return channel is arranged inside the transmission shaft, and the return channel is communicated with the outflow cavity.

In some embodiments, the driven shaft is an optical axis.

In some embodiments, the driven shaft includes a first shaft part and a second shaft part, the first shaft part is located inside the sealing cover, the second shaft part is located outside the sealing cover, a spiral structure is arranged at an outer side of the second shaft part, and a rotation direction of the spiral structure is opposite to a rotation direction of the driven shaft.

In some embodiments, a wear-resistant insert is arranged between the driven shaft and the perfusion cavity body, the wear-resistant insert is fixedly connected with the perfusion cavity body, the wear-resistant insert is rotatable with respect to the driven shaft, the driven shaft is provided with a projection, and the wear-resistant insert is matched with the projection to axially limit the driven shaft and restrict the driven shaft from moving to a distal end of the catheter pump.

In some embodiments, the handle module further includes a first diffusion stress pipe, a motor housing, a second diffusion stress pipe and a cable. One end of the first diffusion stress pipe is connected with the multi-cavity pipe, and an other end of the first diffusion stress pipe is connected with the perfusion cavity body. The first diffusion stress pipe is provided with a perfusion pipe and an outflow pipe. One end of the perfusion pipe is communicated with the perfusion port. One end of the outflow pipe is communicated with the outflow port. The motor housing is arranged at an outer side of a driving motor, the motor housing is detachably connected with the perfusion cavity body, and the motor housing is connected with the perfusion cavity body through a locking nut. One end of the second diffusion stress pipe is connected with the motor housing, an other end of the second diffusion stress pipe is connected with the cable, and one end of the cable is connected with the driving motor.

In some embodiments, the multi-cavity pipe includes an inner pipe and an outer pipe, the inner pipe is coaxially arranged at an inner side of the outer pipe, and the perfusion cavity and a pressure measuring cavity are arranged between the inner pipe and the outer pipe. The pressure measuring cavity is separated from the perfusion cavity by a partition plate. The pressure measuring cavity is not communicated with the perfusion cavity. The transmission shaft is located in the inner pipe, and the outflow cavity is provided between the transmission shaft and the inner pipe. The pressure measuring cavity is communicated with a human body through a pressure measuring hole formed on a side wall of the outer pipe. A detection cavity is arranged in the perfusion cavity body, and the detection cavity is respectively communicated with the pressure measuring cavity and a pressure measuring pipe which is configured to allow pressure measuring liquid to pass through. A pressure measuring element is arranged in the detection cavity, and the pressure measuring element is configured to detect an internal pressure conducted by the pressure measuring liquid.

According to the present invention, the connecting part is a groove and/or a through hole.

In some embodiments, a liquid path channel is arranged in the impeller rotating shaft, the connecting part is the through hole, the liquid path channel is communicated with the through hole, and the hub passes through the through hole and forms a film layer on an inner wall of the impeller rotating shaft.

According to the present invention, the impeller rotating shaft includes a first rigid section, a flexible section and a second rigid section which are sequentially arranged, the connecting part is located in the flexible section, and the flexible section is bendable.

In some embodiments, the flexible section has a hypotube structure.

Compared with the prior art, the following technical effects are derived in the present disclosure.

According to the present disclosure, the impeller structure is bendable in the axial direction, thus reducing the damage of the blood pumping catheter to the blood vessel wall in the pushing process. By means of an integral injection molding of the impeller rotating shaft with the impeller, and through the connecting part on the impeller rotating shaft, the binding force between the impeller and the impeller rotating shaft is increased. In the catheter pump according to the present disclosure, the support is stable, and the same transmission shaft can be used as the impeller rotating shaft and the flexible rotating shaft, thus avoiding radial deflection, reducing the risk that the impeller scrapes with the housing, reducing the occurrence of hemolysis and reducing particles entering the human body. The catheter pump according to the present disclosure is simple in structure, low in cost and high in reliability. The present disclosure enables perfusion liquid to increase the interface lubrication, reduce the friction, and cool down and dissipate heat.

### BRIEF DESCRIPTION OF THE DRAWINGS

In order to explain the embodiments of the present disclosure or the technical solution in the prior art more clearly, the drawings needed in the embodiments will be briefly introduced hereinafter. Apparently, the drawings in the following description are only some embodiments of the present disclosure. Those skilled in the art can obtain other drawings in accordance with these drawings without paying creative labor.
FIG. 1 is a schematic diagram of a catheter pump according to an embodiment of the present disclosure.
FIG. 2 is a schematic diagram of a distal end of a catheter pump according to an embodiment of the present disclosure (having no sheath cover and no switching block).
FIG. 3 is a schematic diagram of the connection between a housing and a multi-cavity pipe according to an embodiment of the present disclosure.
FIG. 4 is a schematic diagram of a multi-cavity pipe according to an embodiment of the present disclosure.
FIG. 5 is a first schematic diagram of a proximal end of a catheter pump according to an embodiment of the present disclosure.
FIG. 6 is a first schematic diagram of the connection between a pigtail pipe and an impeller rotating shaft according to an embodiment of the present disclosure.
FIG. 7 is a second schematic diagram of the connection between a pigtail pipe and an impeller rotating shaft according to an embodiment of the present disclosure.
FIG. 8 is a schematic diagram of an application of an impeller structure according to an embodiment of the present disclosure.
FIG. 9 is a first schematic diagram of an impeller rotating shaft according to an embodiment of the present disclosure.
FIG. 10 is a partial enlarged view of portion I in FIG. 9.
FIG. 11 is a second schematic diagram of an impeller rotating shaft according to an embodiment of the present disclosure.
FIG. 12 is a partial enlarged view of portion II in FIG. 11.
FIG. 13 is a third schematic diagram of an impeller rotating shaft according to an embodiment of the present disclosure.
FIG. 14 is a partial enlarged view of portion III in FIG. 13.
FIG. 15 is a fourth schematic diagram of an impeller rotating shaft according to an embodiment of the present disclosure.
FIG. 16 is a partial enlarged view of portion IV in FIG. 15.
FIG. 17 is a schematic diagram of a hub passing through a through hole according to an embodiment of the present disclosure.
FIG. 18 is a cross-sectional view of an impeller rotating shaft according to an embodiment of the present disclosure.
FIG. 19 is a cross-sectional view of an application of an impeller structure according to an embodiment of the present disclosure.
FIG. 20 is a front view of an impeller rotating shaft according to an embodiment of the present disclosure.
FIG. 21 is a schematic diagram of a bending of an impeller rotating shaft according to an embodiment of the present disclosure.
FIG. 22 is a schematic diagram of the connection between a proximal end of an impeller rotating shaft and a transmission shaft according to an embodiment of the present disclosure.
FIG. 23 is a first schematic diagram of a multi-cavity pipe according to an embodiment of the present disclosure.
FIG. 24 is a schematic diagram of perfusion liquid flowing in a perfusion cavity, a third channel, a liquid path channel and an outflow cavity according to an embodiment of the present disclosure.
FIG. 25 is a schematic diagram of perfusion liquid entering a human body through a liquid path channel according to an embodiment of the present disclosure (an impeller rotating shaft having no rotating shaft step).
FIG. 26 is a schematic diagram of a distal end of a catheter pump according to an embodiment of the present disclosure (having a sheath cover and a switching block).
FIG. 27 is a schematic diagram of the connection between a proximal end of an impeller rotating shaft and a switching block according to an embodiment of the present disclosure.
FIG. 28 is a side view of the connection between a proximal end of an impeller rotating shaft and a switching block according to an embodiment of the present disclosure.
FIG. 29 is a first schematic diagram of sliding between a proximal end of an impeller rotating shaft and a switching block according to an embodiment of the present disclosure.
FIG. 30 is a second schematic diagram of sliding between a proximal end of an impeller rotating shaft and a switching block according to an embodiment of the present disclosure.
FIG. 31 is a schematic diagram of the connection between a proximal end of an impeller rotating shaft and a switching block according to an embodiment of the present disclosure.
FIG. 32 is an external schematic diagram of an impeller rotating shaft, a sheath cover and a transmission shaft according to an embodiment of the present disclosure.
FIG. 33 is an internal schematic diagram of an impeller rotating shaft, a sheath cover and a transmission shaft according to an embodiment of the present disclosure.
FIG. 34 is a partial enlarged view of an impeller rotating shaft, a sheath cover and a transmission shaft according to an embodiment of the present disclosure.
FIG. 35 is a schematic diagram of an expandable bracket at a contracted state according to an embodiment of the present disclosure.
FIG. 36 is a schematic diagram of an expandable bracket at an expanded state according to an embodiment of the present disclosure.
FIG. 37 is a schematic diagram of perfusion liquid flowing in a perfusion cavity, a first channel, a third channel, a liquid path channel and an outflow cavity according to an embodiment of the present disclosure.
FIG. 38 is a schematic diagram of perfusion liquid flowing in a perfusion cavity, a first channel and a liquid path channel according to an embodiment of the present disclosure.
FIG. 39 is a schematic diagram of perfusion fluid flowing in a perfusion cavity and a third channel according to an embodiment of the present disclosure.
FIG. 40 is a schematic diagram of perfusion fluid flowing in a perfusion cavity and an outflow cavity according to an embodiment of the present disclosure.
FIG. 41 is a first schematic diagram of a sealing cover according to an embodiment of the present disclosure.
FIG. 42 is a second schematic diagram of a sealing cover according to an embodiment of the present disclosure.
FIG. 43 is a first schematic diagram of the connection between a sealing cover and a perfusion cavity body according to an embodiment of the present disclosure.
FIG. 44 is a second schematic diagram of the connection between a sealing cover and a perfusion cavity body according to an embodiment of the present disclosure.
FIG. 45 is a schematic diagram of the connection between an internal magnetic component and a driven shaft according to an embodiment of the present disclosure.
FIG. 46 is a first schematic diagram of a driven shaft moving in an axial direction of the driven shaft according to an embodiment of the present disclosure.
FIG. 47 is a second schematic diagram of a driven shaft moving in an axial direction of the driven shaft according to an embodiment of the present disclosure.
FIG. 48 is a schematic diagram of a driven shaft, an internal magnetic component and a sealing cover according to an embodiment of the present disclosure before installation.
FIG. 49 is a schematic diagram of a driven shaft, an internal magnetic component and a sealing cover according to an embodiment of the present disclosure after installation.
FIG. 50 is a schematic diagram of an external magnetic component and an external magnetic component mounting sleeve according to an embodiment of the present disclosure before installation.
FIG. 51 is a schematic diagram of an external magnetic component driving an internal magnetic component to rotate according to an embodiment of the present disclosure.
FIG. 52 is a schematic diagram of an external magnetic component driving an internal magnetic component to rotate according to an embodiment of the present disclosure.
FIG. 53 is a schematic diagram of a flow direction of perfusion liquid in a transmission sealing structure in FIG. 5 according to an embodiment of the present disclosure.
FIG. 54 is a second schematic diagram of a proximal end of a catheter pump according to an embodiment of the present disclosure.
FIG. 55 is a schematic diagram of a flow direction of perfusion liquid in a transmission sealing structure in FIG. 54 according to an embodiment of the present disclosure.
FIG. 56 is a third schematic diagram of a proximal end of a catheter pump according to an embodiment of the present disclosure.
FIG. 57 is a partial enlarged view of a spiral structure according to an embodiment of the present disclosure.
FIG. 58 is a schematic diagram of a driven shaft according to an embodiment of the present disclosure (having a spiral structure).
FIG. 59 is a schematic diagram of a flow direction of perfusion liquid in a transmission sealing structure in FIG. 56 according to an embodiment of the present disclosure.
FIG. 60 is a fourth schematic diagram of a proximal end of a catheter pump according to an embodiment of the present disclosure.
FIG. 61 is a schematic diagram of a flow direction of perfusion liquid in a transmission sealing structure in FIG. 60 according to an embodiment of the present disclosure.
FIG. 62 is a fifth schematic diagram of a proximal end of a catheter pump according to an embodiment of the present disclosure.
FIG. 63 is a schematic diagram of a flow direction of perfusion liquid in a transmission sealing structure in FIG. 62 according to an embodiment of the present disclosure.
FIG. 64 is a second schematic diagram of a multi-cavity pipe according to an embodiment of the present disclosure.
FIG. 65 is a side view of a multi-cavity pipe according to an embodiment of the present disclosure.
FIG. 66 is a sixth schematic diagram of a proximal end of a catheter pump according to an embodiment of the present disclosure.
FIG. 67 is a schematic diagram of a flow direction of perfusion liquid in a transmission sealing structure in FIG. 66 according to an embodiment of the present disclosure.
FIG. 68 is a seventh schematic diagram of a proximal end of a catheter pump according to an embodiment of the present disclosure.
FIG. 69 is a schematic diagram of a flow direction of perfusion fluid in a transmission sealing structure in FIG. 68 according to an embodiment of the present disclosure.
FIG. 70 is a schematic diagram of perfusion liquid entering a human body through a liquid path channel according to an embodiment of the present disclosure (an impeller rotating shaft having a rotating shaft step).
FIG. 71 is a schematic diagram of a hydraulic chamber and a rotating shaft fit gap according to an embodiment of the present disclosure.

Reference numerals in the figures: 1-pigtail pipe, 2-impeller structure, 3-expandable bracket, 4-housing, 5-multi-cavity pipe, 6-handle module, 7-impeller rotating shaft, 8-impeller, 9-hub, 10-blade, 11-liquid path channel, 12-transmission shaft, 13-blood inlet window, 14-blood outlet window, 15-rotating structure, 16-rotating shaft step, 17-groove, 18-through hole, 19-film layer, 20-first rigid section, 21-flexible section, 22-second rigid section, 23-perfusion cavity, 24-outflow cavity, 25-inlet, 26-inner pipe, 27-outer pipe, 28-spring pipe, 29-switching block, 30-first channel, 3 1-protrusion, 32-chute, 33-second channel, 34-sheath cover, 35-opening, 36-sheath cover limiting step, 37-third channel, 38-perfusion cavity body, 39-pressure measuring hole, 40-driving motor, 41-external magnetic component, 42-internal magnetic component, 43-driven shaft, 44-sealing cover, 45-proximal end insert, 46-external magnetic component mounting sleeve, 47-opening end, 48-sealing end, 50-first shaft part, 51-second shaft part, 52-spiral structure, 53-wear-resistant insert, 54-projection, 55-first diffusion stress pipe, 56-motor housing, 57-second diffusion stress pipe, 58-cable, 59-perfusion pipe, 60-outflow pipe, 61-locking nut, 62-pressure measuring cavity, 63-partition plate, 64-detection cavity, 65-pressure measuring pipe, 66-pressure measuring element, 67-inner ring of a bearing, 68-outer ring of a bearing, 70-first connecting part, 71-second connecting part, 72-plug, 73-shaft sleeve, 74-transmission line, 75-transmission line antenna, 76-hydraulic chamber, 77-rotating shaft fit gap.

### DETAILED DESCRIPTION OF THE EMBODIMENTS

The technical solution in the embodiment of the present disclosure will be described clearly and completely with reference to the drawings in the embodiment of the present disclosure. Apparently, the described embodiments are only some embodiments of the present disclosure, rather than all of the embodiments. Based on the embodiments in the present disclosure, all other embodiments obtained by those skilled in the art without paying creative labor fall within the scope of protection of the present disclosure.

The purpose of the present disclosure is to provide a catheter pump, which solves the problem in the prior art that a poor binding force between the rotating shaft and the impeller by means of the integral injection molding of the rotating shaft and the impeller.

In order to make the above purpose, features and advantages of the present disclosure more obvious and understandable, the present disclosure will be further described in detail with reference to the drawings and the detailed description.

### Embodiment 1

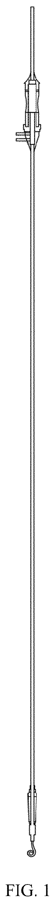

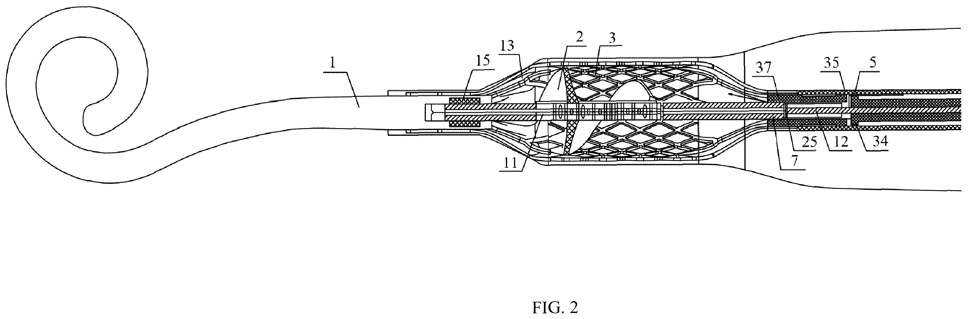

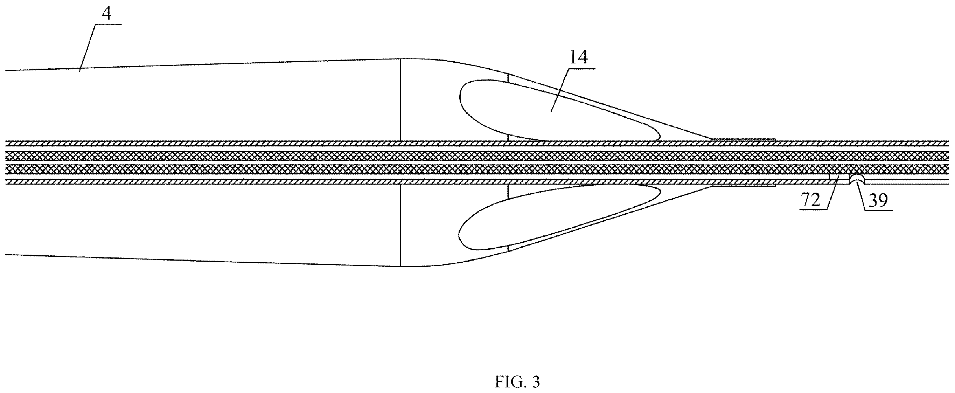

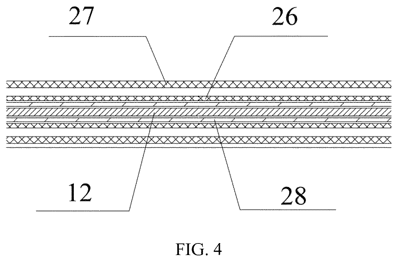

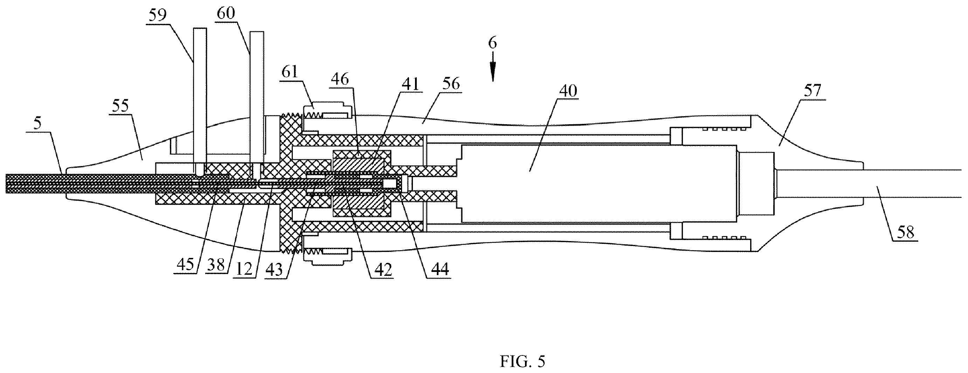

As shown in FIG. 1 to FIG. 5, this embodiment provides a catheter pump, which includes a pigtail pipe 1, a functional module, an expandable bracket 3, a housing 4, a liquid path module and a handle module 6. The functional module includes an impeller structure 2. The impeller structure 2 includes an impeller rotating shaft 7 and an impeller 8. The impeller rotating shaft 7 is configured to transmit a torque and drive the impeller 8 to rotate. The impeller 8 includes a hub 9 and several blades 10. The several blades 10 are arranged on the hub 9. A liquid path channel 11 is arranged inside the impeller rotating shaft 7. The liquid path channel 11 penetrates from a proximal end of the impeller rotating shaft 7 to a distal end of the impeller rotating shaft 7. A side wall of the impeller rotating shaft 7 is provided with a connecting part. The hub 9 is connected with the connecting part by integral injection molding. The connecting part is configured to limit relative movement of the impeller 8. The impeller rotating shaft 7 is rotatably connected with the pigtail pipe 1. The liquid path module includes a multi-cavity pipe 5. The impeller rotating shaft 7 is connected with a driving motor 40 in the handle module 6 through a transmission shaft 12 in the multi-cavity pipe 5. The expandable bracket 3 is located at an outer side of the impeller structure 2. A distal end of the expandable bracket 3 is connected with the pigtail pipe 1. A proximal end of the expandable bracket 3 is connected with the multi-cavity pipe 5. The housing 4 is located at an outer side of the expandable bracket 3. The housing 4 is connected with the pigtail pipe 1 and the multi-cavity pipe 5, respectively, and the housing 4 is provided with a blood inlet window 13 and a blood outlet window 14. The blood inlet window 13 is provided near the distal end of the catheter pump, and the blood outlet window 14 is provided near the proximal end of the catheter pump.

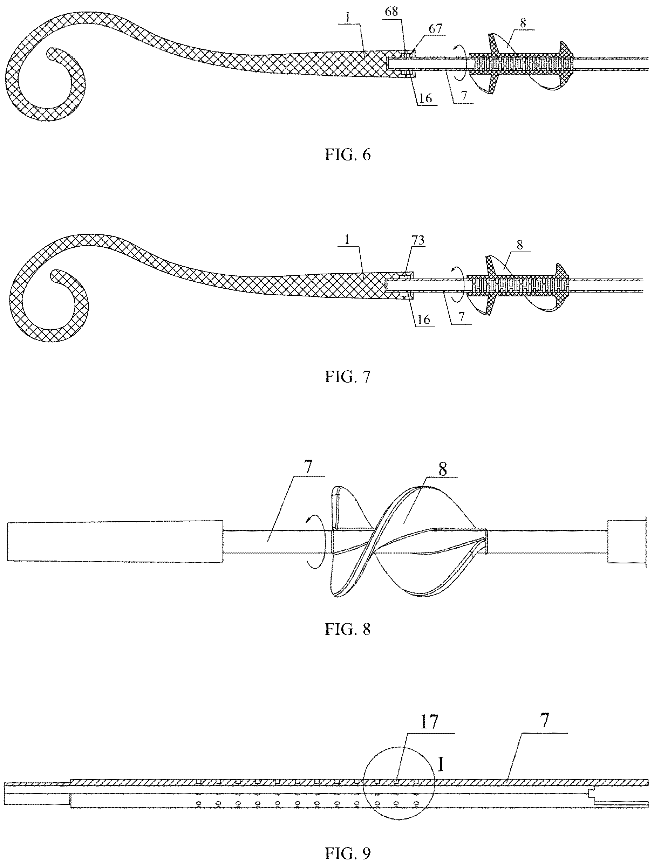

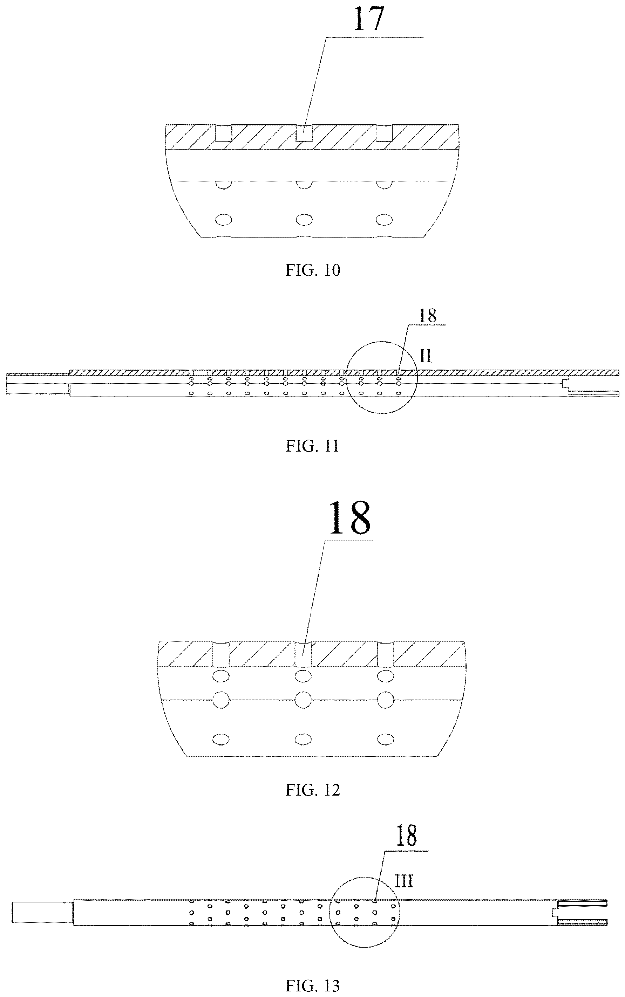

Specifically, as shown in FIG. 9 and FIG. 10, in this embodiment, the connecting part is a groove 17. The portion of the hub 9 corresponding to the groove 17 is located in the groove 17 and attached to the surface of the groove 17, so that the movement of the impeller 8 can be restricted. Compared with the impeller rotating shaft 7 with a smooth surface in the prior art, in this embodiment, the hub 9 is bound with the groove 17, increasing the binding force between the impeller 8 and the impeller rotating shaft 7. Moreover, because the connecting part is the groove 17, the area where the connecting part is located can have a certain bending deflection.

In this embodiment, the impeller 8 is made of a polymer material, in other embodiments is made of one of a natural rubber, an isoprene rubber or thermoplastic polyurethane.

In this embodiment, the impeller rotating shaft 7 is made of a metal material, in other embodiments is made of one of a cobalt-chromium alloy, a nickel-titanium alloy, a stainless steel (304 stainless steel or 316 stainless steel) or a tungsten alloy. The impeller rotating shaft 7 can be hardened by DLC or surface nitriding.

In this embodiment, the connecting part is provided, so that the binding force between the impeller rotating shaft 7 and the impeller 8 is improved, avoiding a relative movement between the impeller 8 and the impeller rotating shaft 7, avoiding a relative rotation between the impeller 8 and the impeller rotating shaft 7, so that the working efficiency of the impeller 8 is improved, and the risk that the impeller 8 scrapes with the housing 4 is avoided, reducing the occurrence of hemolysis, and reducing particles entering the body.

In this embodiment, the distal end of the impeller rotating shaft 7 is rotatably connected with the proximal end of the pigtail pipe 1 through a rotating structure 15. The rotating structure 15 includes two types of structures. As shown in FIG. 7, the first rotating structure 15 is a shaft sleeve 73. The shaft sleeve 73 is made of ceramics. The distal end of the impeller rotating shaft 7 is located at the inner side of the shaft sleeve 73 and is fixed with the shaft sleeve 73. As shown in FIG. 6, the second rotating structure 15 is a bearing, in other embodiments, the second rotating structure 15 is a ball bearing. The distal end of the impeller rotating shaft 7 is located at the inner side of the inner ring 67 of the bearing and is fixed with the inner ring 67 of the bearing. The pigtail pipe 1 is located at an outer side of the outer ring 68 of the bearing and is fixed with the outer ring 68 of the bearing. If the impeller rotating shaft 7 is fixed with the inner ring 67 of the bearing, the impeller rotating shaft 7 is axially fixed with respect to the pigtail pipe 1. If the impeller rotating shaft 7 is matched with the shaft sleeve 73, the impeller rotating shaft 7 can move axially with respect to the pigtail pipe 1. The distal end of the impeller rotating shaft 7 is provided with a rotating shaft step 16. The proximal end of the rotating structure 15 is matched with the rotating shaft step 16 to axially limit the impeller rotating shaft 7 and restrict the impeller rotating shaft 7 from moving to the distal end of the catheter pump.

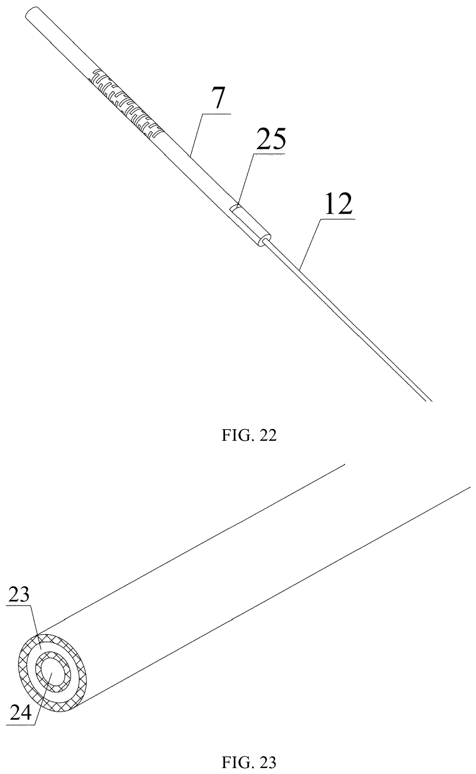

In this embodiment, as shown in FIG. 23, the multi-cavity pipe 5 is made of a plastic material such as polyamide and Pebax. The multi-cavity pipe 5 includes an inner pipe 26 and an outer pipe 27. The inner pipe 26 is coaxially arranged at an inner side of the outer pipe 27. The perfusion cavity 23 is arranged between the inner pipe 26 and the outer pipe 27. The transmission shaft 12 is coaxially arranged in the inner pipe 26. The outflow cavity 24 is formed between the transmission shaft 12 and the inner pipe 26. The transmission shaft 12 is configured to transmit a driving torque. The transmission shaft 12 is made of a stainless steel or a Co-Cr-Mo alloy. A spring pipe 28 is arranged between the transmission shaft 12 and the inner pipe 26. The outer wall of the spring pipe 28 is in contact with or not in contact with the inner wall of the inner pipe 26. The inner wall of the spring pipe 28 is in contact with or not in contact with the outer wall of the transmission shaft 12. The spring pipe 28 is made of a stainless steel. The spring pipe 28 is configured to restrict the radial runout of the transmission shaft 12 and reduce the running vibration and the noise. The perfusion cavity 23 extends from the proximal end of the multi-cavity pipe 5 to the distal end of the multi-cavity pipe 5. The outflow cavity 24 extends from the proximal end of the multi-cavity pipe 5 to the distal end of the multi-cavity pipe 5. The distal end of the outflow cavity 24 is communicated with the distal end of the perfusion cavity 23. The proximal end of the impeller rotating shaft 7 is connected with the distal end of the transmission shaft 12. As shown in FIG. 22, an inlet 25 communicated with the liquid path channel 11 is formed on the impeller rotating shaft 7. The inlet 25 is a through-hole structure formed on the side wall of the impeller rotating shaft 7. The inlet 25 is communicated with the perfusion cavity 23, the liquid path channel 11 and the outflow cavity 24, respectively. Perfusion fluid flows from the perfusion cavity 23, flows into the liquid path channel 11 from the proximal end of the impeller rotating shaft 7 through the inlet 25, flows along the liquid path channel 11 to the distal end of the impeller rotating shaft 7, flows out from a gap between the impeller rotating shaft 7 and the rotating structure 15, and then enters the human body. Meanwhile, the perfusion fluid flows out from the outflow cavity 24. Perfusion liquid flowing in the catheter pump can increase the interface lubrication, reduce the friction, and cool down and dissipate heat.

In this embodiment, a sheath cover 34 is arranged at an outer side of a joint between the proximal end of the impeller rotating shaft 7 and the distal end of the transmission shaft 12. The sheath cover 34 can be made of the same material as the impeller rotating shaft 7 or the ceramic material. There is a gap between the impeller rotating shaft 7 and the inner side of the sheath cover 34. The fit gap is 1 um-8 um, in other embodiments, the fit gap is 1 um-3 um, so that the impeller rotating shaft 7 can rotate with respect to the sheath cover 34. Both ends of the sheath cover 34 are connected with the expandable bracket 3 and the multi-cavity pipe 5, respectively. The sheath cover 34 is provided with an opening 35. The opening 35 enables the distal end of the perfusion cavity 23 to be communicated with the distal end of the outflow cavity 24.

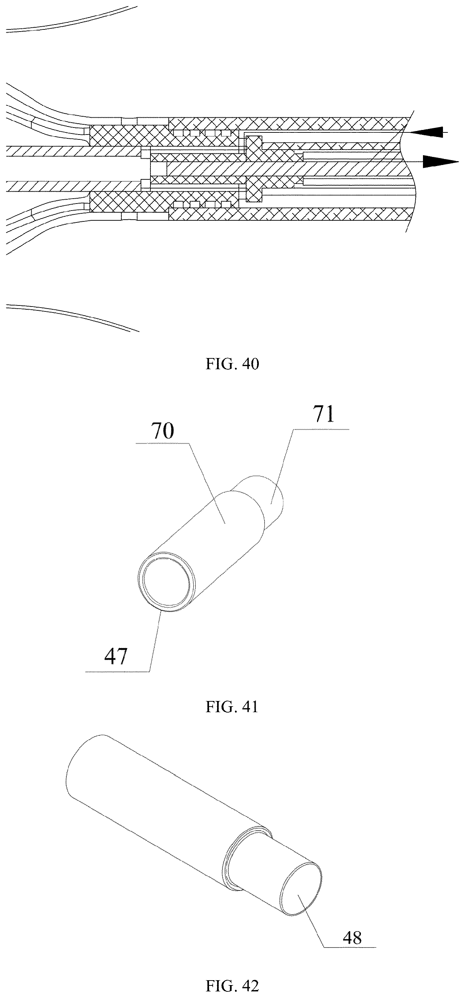

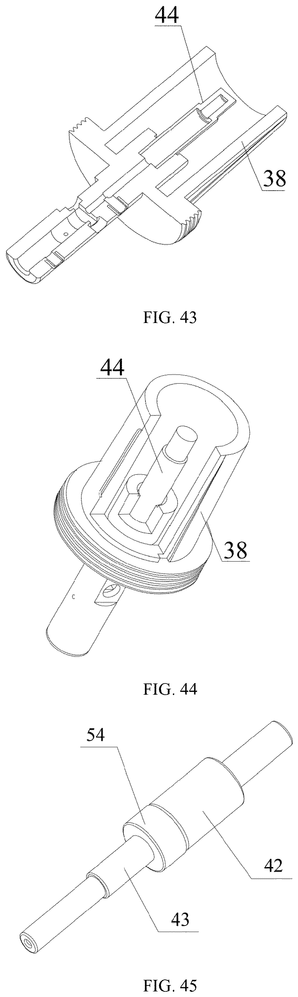

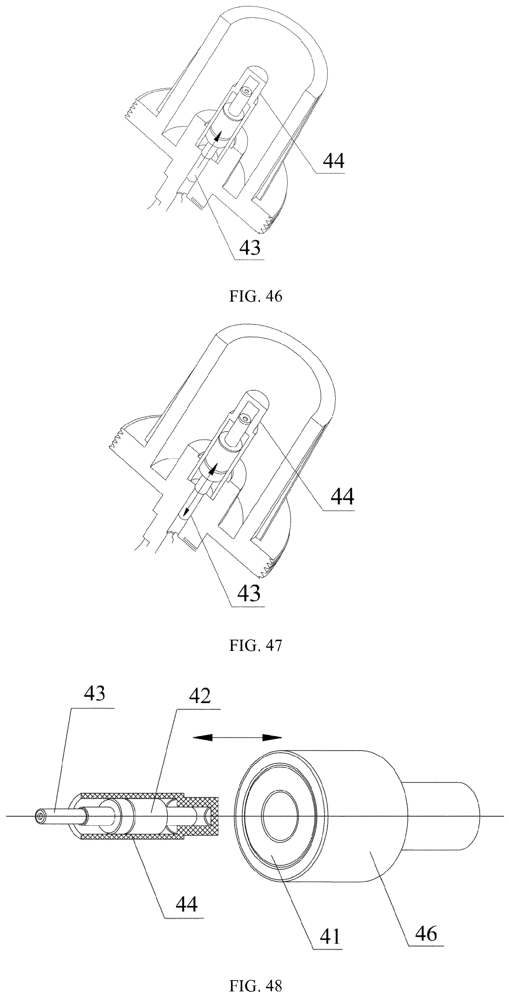

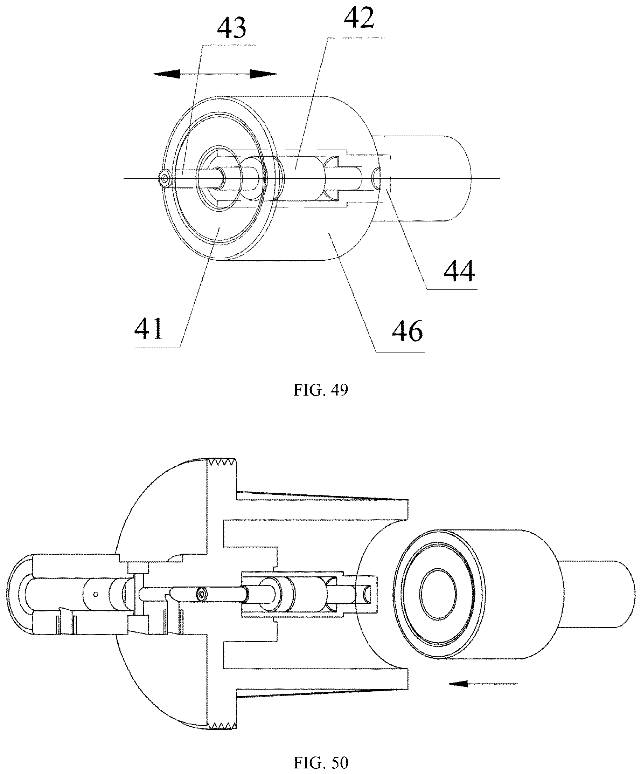

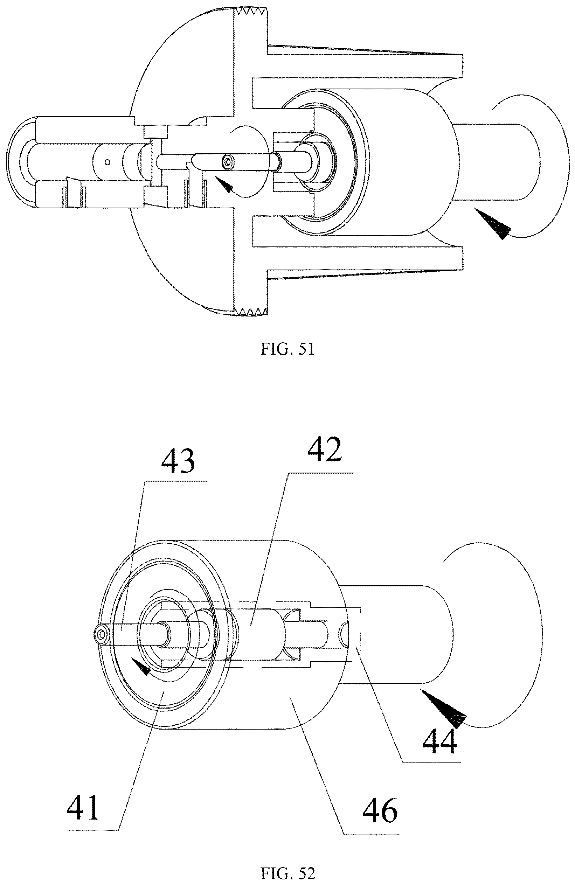

In this embodiment, as shown in FIG. 41 to FIG. 52, the handle module 6 includes a perfusion cavity body 38, a transmission sealing structure, a driving motor 40 and a proximal end insert 45. The perfusion cavity 23 of the multi-cavity pipe 5 is communicated with the perfusion port of the perfusion cavity body 38. The outflow cavity 24 of the multi-cavity pipe 5 is communicated with the outflow port of the perfusion cavity body 38. The proximal end insert 45 is made of a metal material such as a stainless steel. The proximal end insert 45 is sleeved at the outer side of the transmission shaft 12, and there is a gap between the proximal end insert 45 and the transmission shaft 12. The proximal end of the proximal end insert 45 is fixedly connected with the perfusion cavity body 38. The distal end of the proximal end insert 45 is hermetically connected with the inner cavity of the multi-cavity pipe 5. The proximal end insert 45 plays a role in separating the outflow cavity 24 from the perfusion cavity 23, so that the proximal end of the multi-cavity pipe 5 is connected with the perfusion cavity body 38, and the proximal end of the perfusion cavity 23 is not communicated with the proximal end of the outflow cavity 24. The transmission sealing structure includes an external magnetic component 41, an internal magnetic component 42, a driven shaft 43 and a sealing cover 44. The external magnetic component 41, the internal magnetic component 42, the driven shaft 43 and the sealing cover 44 are all coaxially arranged. Both the internal magnetic component 42 and the external magnetic component 41 are made of neodymium iron boron and other materials. Both the internal magnetic component 42 and the external magnetic component 41 include a magnetic ring. The internal magnetic component 42 is sleeved on the driven shaft 43 and is adhered with the driven shaft 43. The internal magnetic component 42 is configured to receive a magnetic torque. The sealing cover 44 is arranged at an outer side of the internal magnetic component 42. The external magnetic component 41 is arranged on an external magnetic component mounting sleeve 46. The external magnetic component mounting sleeve 46 is located at the inner side of the perfusion cavity body 38. The external magnetic component mounting sleeve 46 is fixedly connected with the external magnetic ring and the output shaft of the driving motor 40, respectively. The external magnetic component mounting sleeve 46 is configured to transmit the rotating torque of the output shaft of the driving motor 40 and fix the external magnetic ring. The external magnetic component mounting sleeve 46 is made of engineering plastics such as Peek, PC, and ABC. The external magnetic component 41 is located at an outer side of the sealing cover 44. There is a gap between the external magnetic component 41 and the sealing cover 44. The sealing cover 44 is made of non-metallic materials such as ceramics (such as alumina and zirconia), glass, and diamond. Alternatively, the sealing cover 44 is made of plastic, and a non-metallic wear-resistant coating is arranged outside the plastic, so that the opening end 47 of the sealing cover 44 is hermetically connected with the perfusion cavity body 38 in a manner of adhesion. The outer wall of the sealing cover 44 is hermetically connected with the perfusion cavity body 38 in a manner of interference fit. The sealing cover 44 is configured to isolate the internal magnetic component 42 from the external magnetic component 41. The rotation of the external magnetic component 41 in turn drives both the internal magnetic component 42 and the driven shaft 43 to rotate with respect to the sealing cover 44. The driven shaft 43 and the internal magnetic component 42 can also move in the axial direction of the sealing cover 44.

In this embodiment, the handle module 6 further includes a first diffusion stress pipe 55, a motor housing 56, a second diffusion stress pipe 57 and a cable 58. The first diffusion stress pipe 55 is made of elastic materials such as rubbers, silica gel or polyurethane. The distal end of the first diffusion stress pipe 55 is fixedly connected with the multi-cavity pipe 5. The proximal end of the first diffusion stress pipe 55 is fixedly connected with the perfusion cavity body 38. The first diffusion stress pipe 55 is configured to reduce the stress concentration between the multi-cavity pipe 5 and the perfusion cavity body 38, and reduce the folding. The first diffusion stress pipe 55 is provided with a perfusion pipe 59 and an outflow pipe 60. The perfusion pipe 59 is made of materials such as PVC and polyurethane. One end of the perfusion pipe 59 is communicated with the perfusion port. The perfusion pipe 59 is configured to deliver perfusion liquid (heparin physiological saline) into the body. The outflow pipe 60 is made of materials such as PVC and polyurethane. One end of the outflow pipe 60 is communicated with the outflow port. The outflow pipe 60 is configured to receive liquid refluxed in the body and allow the liquid to flow back to the waste liquid bag outside the body. The motor housing 56 is arranged at an outer side of the driving motor 40. The motor housing 56 is detachably connected with the perfusion cavity body 38, and the motor housing 56 is connected with the perfusion cavity body 38 through a locking nut 61. The locking nut 61 is made of ABS/PC. The second diffusion stress pipe 57 is located at an outer side of the driving motor 40 and at an inner side of the housing of the driving motor 40. The distal end of the second diffusion stress pipe 57 is connected with the motor housing 56. The proximal end of the second diffusion stress pipe 57 is connected with the cable 58. One end of the cable 58 is connected with the drive motor 40. The cable 58 is configured to provide power to the motor and transmit the control and detection signals. The second diffusion stress pipe 57 is made of elastic materials such as rubbers, silica gel or polyurethane. The second diffusion stress pipe 57 is configured to reduce the running vibration and the noise of the motor.

In this embodiment, the sealing cover 44 is integrally molded. The sealing cover 44 includes a first connecting part 70 and a second connecting part 71. One end of the first connecting part 70 is an opening end 47, and the other end of the first connecting part 70 is connected with one end of the second connecting part 71. The other end of the second connecting part 71 is a closed end. The driven shaft 43 is provided with a projection 54. The projection 54 is rotatably connected the first connecting part 70 of the sealing cover 44. The second connecting part 71 of the sealing cover 44 is rotatably connected the driven shaft 43.

In this embodiment, the driven shaft 43 has a solid structure. The solid structure is formed by winding a plurality of steel cables. The distal end of the driven shaft 43 is connected with the proximal end of the transmission shaft 12. Alternatively, the driven shaft 43 is of a hollow structure. The hollow structure is a hollow torque spring formed by weaving a plurality of steel cables. The proximal end of the transmission shaft 12 extends into the driven shaft 43. The transmission shaft 12 is a solid shaft.

In this embodiment, the distal end of the driven shaft 43 protrudes from the opening end 47 of the sealing cover 44, and there is a gap between the driven shaft 43 and the perfusion cavity body 38. The gap between the driven shaft 43 and the perfusion cavity body 38 can be communicated with the outflow cavity 24. The proximal end of the driven shaft 43 is close to the sealing end 48 of the sealing cover 44. There are gaps between the driven shaft 43 and the sealing cover 44, and between the internal magnetic component 42 and the sealing cover 44.

When the transmission sealing structure of this embodiment is used, the driving motor 40 drives the external magnetic component mounting sleeve 46 to drive the external magnetic component 41 to rotate. Through the magnetic cooperation between the external magnetic component 41 and the internal magnetic component 42, the external magnetic component 41 drives the internal magnetic component 42 and the driven shaft 43 to rotate. The driven shaft 43 drives the transmission shaft 12 to rotate. The transmission shaft 12 drives the impeller rotating shaft 7 and the impeller 8 to rotate.

The sealing cover 44 of this embodiment can not only achieve the sealing function, but also achieve the rotatable connection, so as to achieve the integrated design of sealing and rotatable connection. In the transmission process, the external magnetic component 41 is not in contact with the internal magnetic component 42. Moreover, the driven shaft 43 can not only rotate, but also move in the axial direction of the driven shaft 43. The transmission sealing structure of this embodiment is simple, high in reliability, long in service life and low in cost.

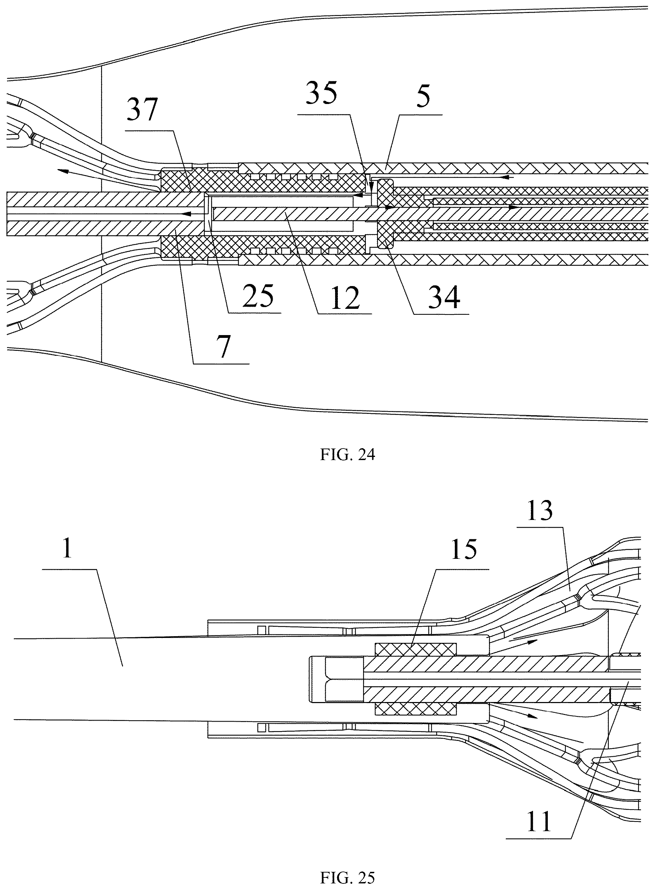

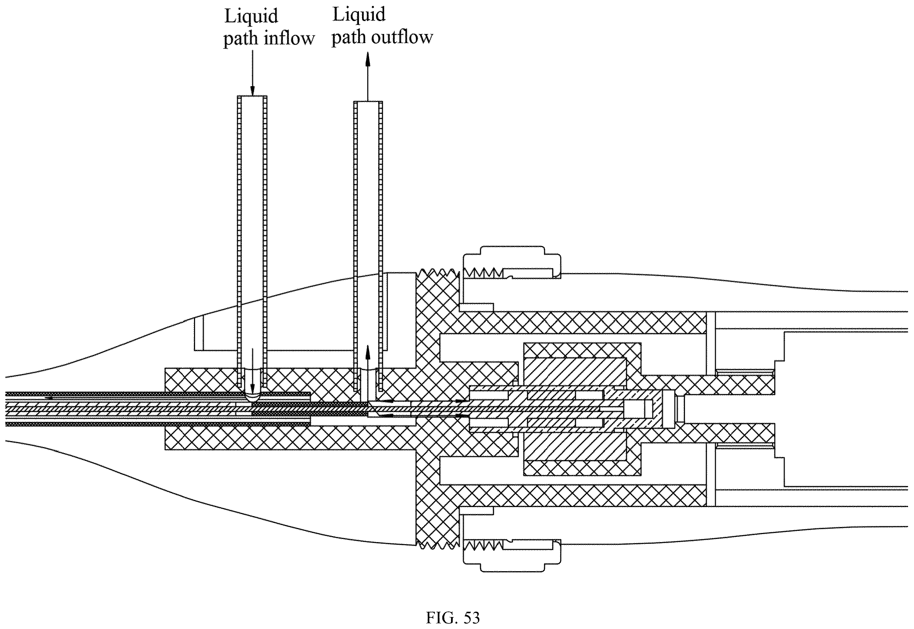

In this embodiment, as shown in FIG. 24, FIG. 70, FIG. 71 and FIG. 53, perfusion liquid enters the perfusion cavity 23 through the perfusion pipe 59 and then is divided into two paths. The perfusion liquid in the first path enters the liquid path channel 11 through the inlet 25, flows to the distal end of the impeller rotating shaft 7, flows out from the gap between the impeller rotating shaft 7 and the rotating structure 15, and then enters the human body. The perfusion liquid in the second path flows out of the outflow cavity 24 and then is divided into two paths including a third path and a forth path. The perfusion liquid in the third path flows into the waste liquid bag through the outflow pipe 60, and the perfusion liquid in the forth path flows to the space formed by the driven shaft 43, the internal magnetic component 42 and the sealing cover 44 through the gap between the driven shaft 43 and the perfusion cavity body 38, and the perfusion liquid flows out from the gap between the driven shaft 43 and the perfusion cavity body 38, and then is discharged through the outflow pipe 60.

### Embodiment 2

The difference between this embodiment and Embodiment 1 is that, as shown in FIG. 25, in this embodiment, there is no rotating shaft step 16 at the distal end of the impeller rotating shaft 7, and the sheath cover 34 may not be provided with the sheath cover limiting step 36. The proximal end of the impeller rotating shaft 7 is fixedly connected with the distal end of the transmission shaft 12. When the expandable bracket 3 changes from the contracted state to the expanded state, the distal end of the expandable bracket 3 drives the pigtail pipe 1, which in turn drives the rotating structure 15 to move to the proximal end with respect to the impeller rotating shaft 7. In this case, the sheath cover limiting step 36 does not play a limiting role. Because the impeller rotating shaft 7 is fixedly connected with the transmission shaft 12 and the proximal end of the transmission shaft 12 is fixedly connected with the driven shaft 43, the sealing end 48 of the sealing cover 44 plays a restriction role to the proximal end of the driven shaft 43, thereby restricting the impeller rotating shaft 7 from moving excessively to the proximal end.

### Embodiment 3

In order to solve the problem that the impeller rotating shaft 7 in the prior art cannot be bent, in this embodiment, the impeller rotating shaft 7 includes a first rigid section 20, a flexible section 21 and a second rigid section 22 which are sequentially arranged. The impeller 8 and the connecting part are both located in the flexible section 21, the connecting part is a through hole 18, and the flexible section 21 is bendable.

In this embodiment, the flexible section 21 has a hypotube structure. The hypotube structure can use the hypotube structure in the prior art. The flexible section 21 of this embodiment enables the impeller structure 2 to be bendable in the axial direction, which reduces the damage of the blood pumping catheter to the blood vessel wall in the pushing process, and solves the problem that the existing impeller rotating shaft 7 cannot be bent.

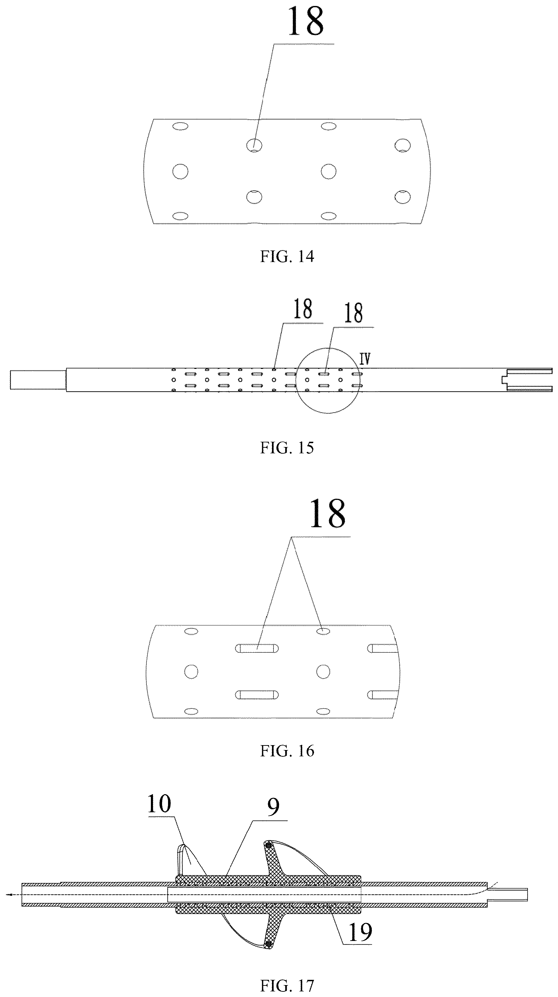

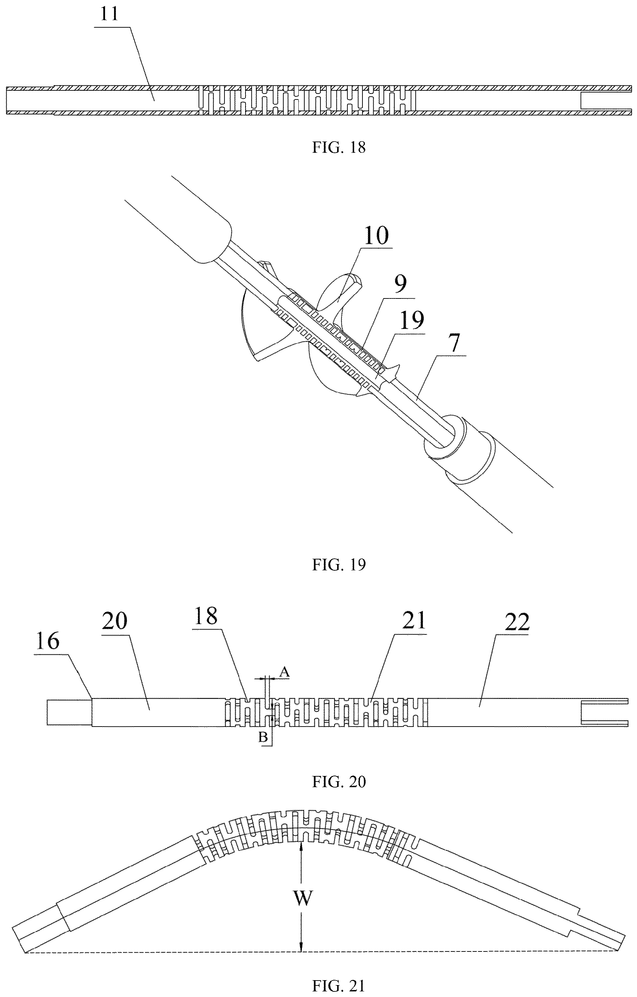

As shown in FIG. 17 and FIG. 19, in this embodiment, the liquid path channel 11 is communicated with the through hole 18. The hub 9 passes through the through hole 18 and forms a film layer 19 on an inner wall of the impeller rotating shaft 7, so that the bonding force between the impeller 8 and the impeller rotating shaft 7 is further increased. The hub 9 covers the inner wall and the outer wall of the impeller rotating shaft 7 where the through hole 18 is located. The part of the hub 9 on the inner wall of the impeller rotating shaft 7 is in contact with the inner wall of the impeller rotating shaft 7. The part of the hub 9 on the outer wall of the impeller rotating shaft 7 is in contact with the outer wall of the impeller rotating shaft 7. The part of the hub 9 in the through hole 18 is in contact with the side wall of the through hole 18 to seal the through hole 18 and prevent the perfusion liquid from leaking from the through hole. The arrangement of the through hole 18 further improves the bending deflection of the impeller rotating shaft 7.

When the impeller rotating shaft 7 and the impeller 8 are integrally injection molded, first, the cut impeller rotating shaft 7 is placed in the mold cavity for positioning. Second, a shaft center with a cylindrical and stepped shape protrudes into the liquid path channel 11 from both ends of the impeller rotating shaft 7. The gap between the shaft center and the inner wall of the impeller rotating shaft 7 is configured to form the part of the hub 9 on the inner wall of the impeller rotating shaft 7, i.e., the film layer 19. Finally, the mold cavity is removed to obtain the impeller structure 2.

### Embodiment 4

In this embodiment, the specific structure of the hypotube structure in Embodiment 3 is defined. As shown in FIG. 18, in this embodiment, the side wall of the flexible section 21 is provided with several circles of through-hole structures in the axial direction of the flexible section 21. The through-hole structure of each circle includes several through-holes 18 arranged in the circumferential direction of the flexible section 21. The through-holes 18 are discontinuous in the circumferential direction of the flexible section 21 so as to maintain the torque transmission of the impeller rotating shaft 71. The through-holes 18 of adjacent circles are arranged in a staggered manner, and the shape of the through-holes 18 is not limited.

### Embodiment 5

As shown in FIG. 20 and FIG. 21, further limitations are made in this embodiment on the basis of the Embodiment 4. In this embodiment, the through holes 18 are long holes. The size B of the connecting part between adj acent through holes 18 of the through-hole structures of the same circle is 0.05 mm-2 mm, preferably 0.1 mm-0.5 mm, more preferably 0.25 mm. The size A of the through hole 18 in the axial direction of the flexible section 21 is 0.1 mm-0.5 mm, preferably 0.1 mm-0.5 mm, and more preferably 0.25 mm. In this embodiment, the maximum bending deflection W of the impeller rotating shaft 7 is in the range of 0.1 mm-5 mm.

In this embodiment, the existence of the through hole 18 can not only improve the binding force between the impeller 8 and the impeller rotating shaft 7, but also increase the bending deflection of the impeller rotating shaft 7.

### Embodiment 6

As shown in FIG. 11 to FIG. 12, in this embodiment, the through holes 18 of adjacent circles are arranged in a manner of one-to-one correspondence, and the through holes 18 are round holes.

### Embodiment 7

As shown in FIG. 13 to FIG. 14, the difference between this embodiment and Embodiment 6 is that in this embodiment, the through holes 18 are round holes, and the through holes 18 of adjacent circles are arranged in a staggered manner.

### Embodiment 8

As shown in FIG. 15 to FIG. 16, in this embodiment, the through holes 18 include round holes and long holes. Each through hole 18 of the through-hole structures of the same circle is a round hole. Each through hole 18 of the through-hole structure adjacent thereto is a long hole, and the through holes 18 of the adjacent circles are arranged in a staggered manner.

### Embodiment 9

The difference between this embodiment and Embodiment 1 is that in this embodiment, the connecting part is a groove 17 and a through hole 18.

### Embodiment 10

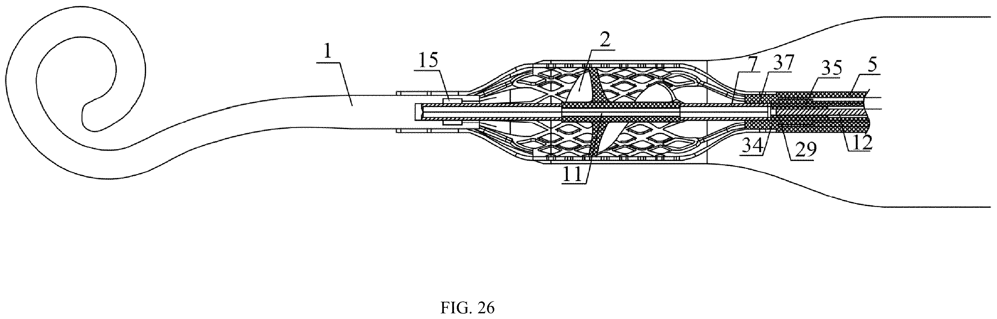

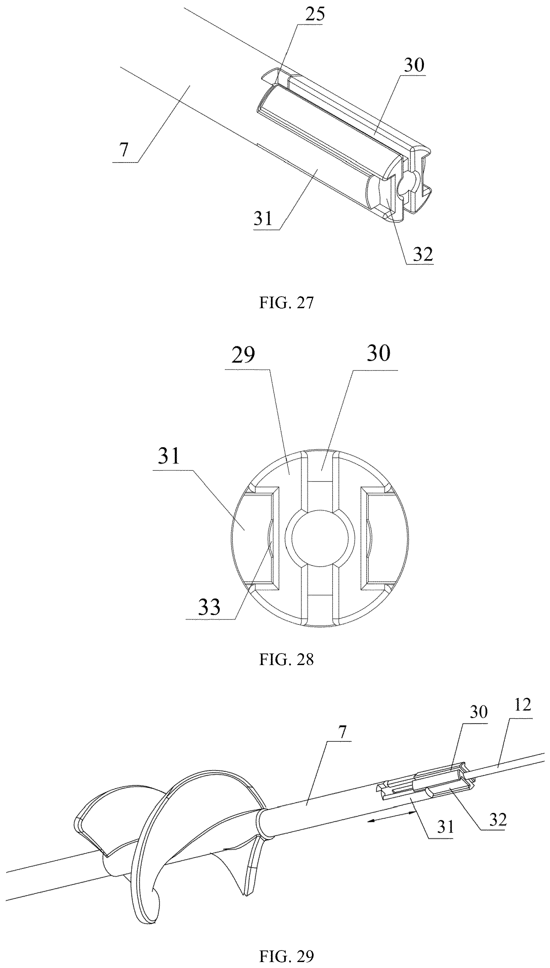

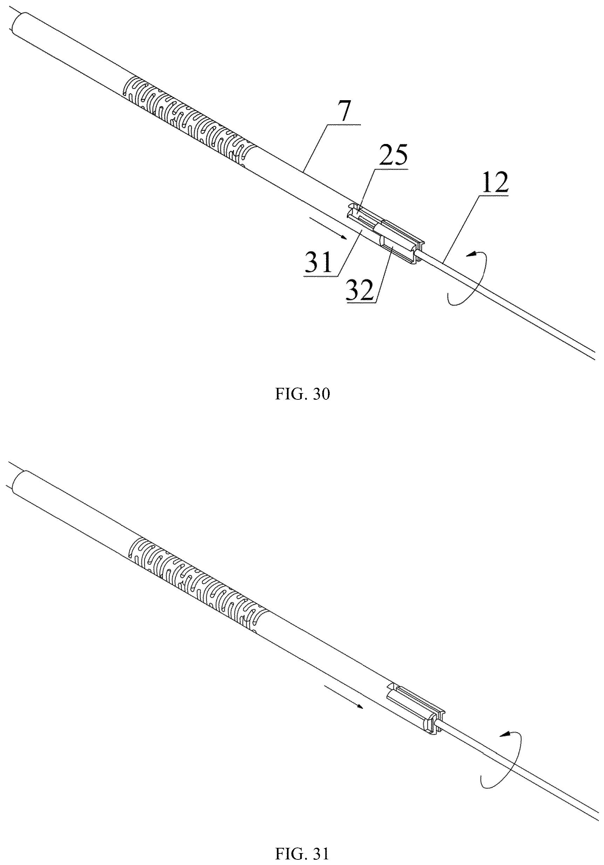

As shown in FIG. 26 to FIG. 31, this embodiment is further optimized on the basis of Embodiment 1. A switching block 29 is included in this embodiment. The switching block 29 can be made of the same material as the impeller rotating shaft 7 or the ceramic material. The proximal end of the impeller rotating shaft 7 is connected with the distal end of the transmission shaft 12 through the switching block 29. In this embodiment, the proximal end of the impeller rotating shaft 7 is provided with protrusions 31. The inlet 25 is arranged between adjacent protrusions 31. The inlet 25 has a notched structure. The switching block 29 is provided with a first channel 30. The first channel 30 is communicated with the inlet 25, the perfusion cavity 23 and the outflow cavity 24, respectively.

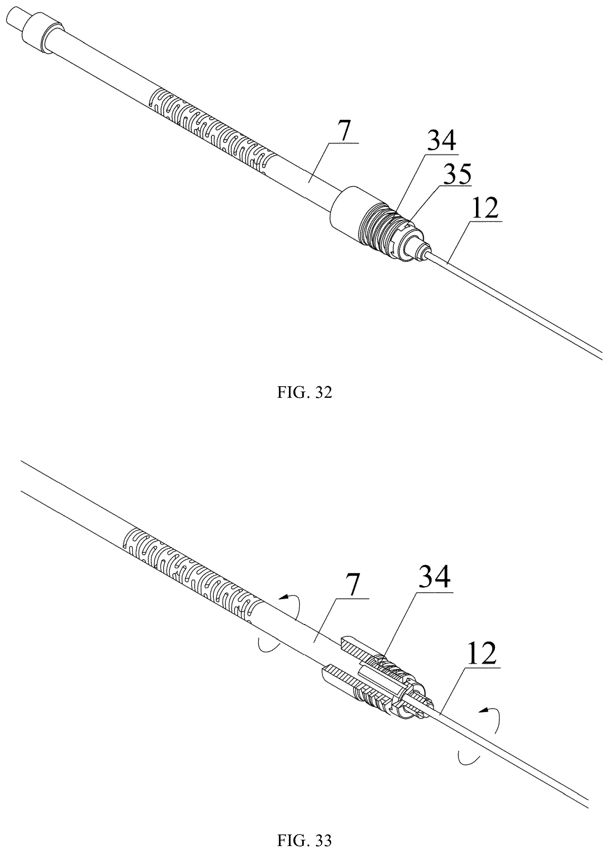

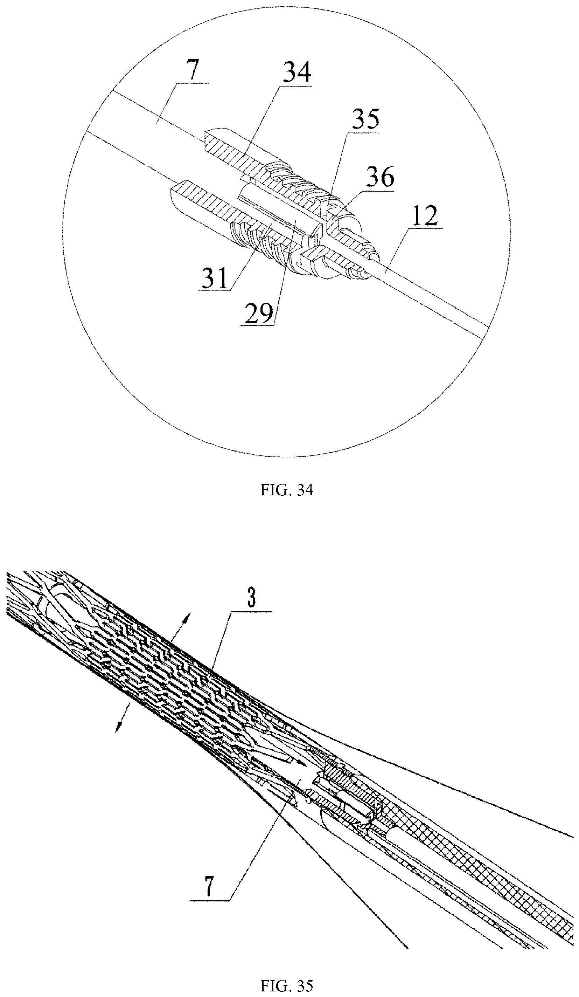

In this embodiment, as shown in FIG. 32 to FIG. 36, a sheath cover 34 is arranged at the outer side of the joint between the proximal end of the impeller rotating shaft 7 and the switching block 29. There is a gap between the outer side of the joint (between the proximal end of the impeller rotating shaft 7 and the switching block 29) and the inner side of the sheath cover 34. The fit gap is 1 um-8 um, preferably 1 um-3 um, so that the joint between the proximal end of the impeller rotating shaft 7 and the switching block 29 is rotatable with respect to the sheath cover 34. The sheath cover 34 is provided with a sheath cover limiting step 36. The impeller rotating shaft 7 is provided with a rotating shaft step 16. The proximal end of the impeller rotating shaft 7 is in axial sliding fit with the switching block 29. The switching block 29 is fixedly connected with the transmission shaft 12. When the expandable bracket 3 changes from the contracted state to the expanded state, the distal end of the expandable bracket 3 drives the pigtail pipe 1, which in turn drives the rotating structure 15. The rotating structure 15 drives the impeller rotating shaft 7. The impeller rotating shaft 7 drives the switching block 29 to move to the proximal end. The sheath cover limiting step 36 restricts the switching block 29 from moving excessively to the proximal end.

In this embodiment, the switching block 29 is provided with a chute 32. The protrusion 31 is in sliding connection with the chute 32. The gap between the inner surface of the protrusion 31 and the surface of the chute 32 is 0.1 mm-0.5 mm. A second channel 33 is formed between the inner surface of the protrusion 31 and the surface of the chute 32. The second channel 33 is communicated with the perfusion cavity 23, the liquid path channel 11 and the outflow cavity 24, respectively.

In this embodiment, the perfusion liquid is divided into three paths after the perfusion liquid enters from the perfusion cavity 23. The perfusion liquid in the first path enters the liquid path channel 11 through the first channel 30 and the inlet 25; meanwhile, the perfusion liquid in the second path enters the liquid path channel 11 through the second channel 33, and the perfusion liquid flows to the distal end of the impeller rotating shaft 7, flows out from the gap between the impeller rotating shaft 7 and the rotating structure 15, and then enters the human body; the perfusion liquid in third path flows out from the outflow cavity 24.

### Embodiment 11

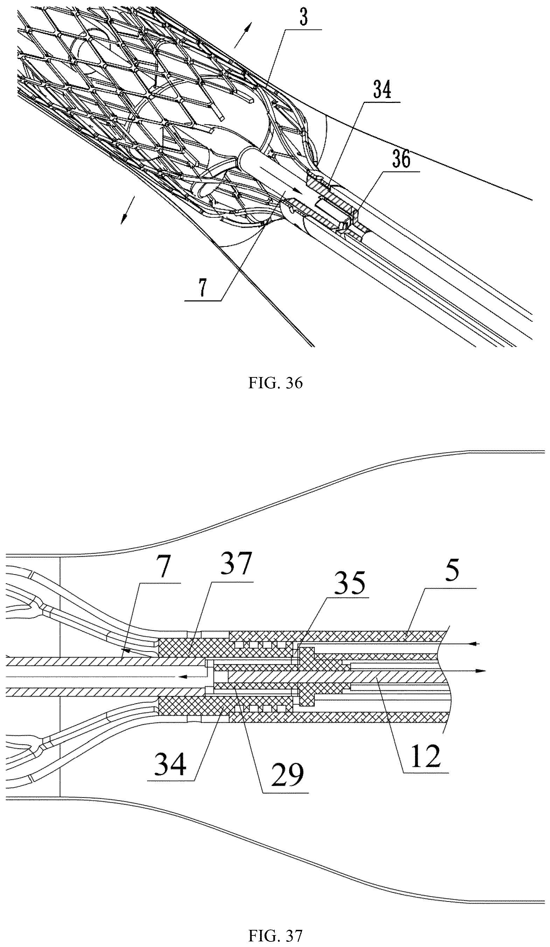

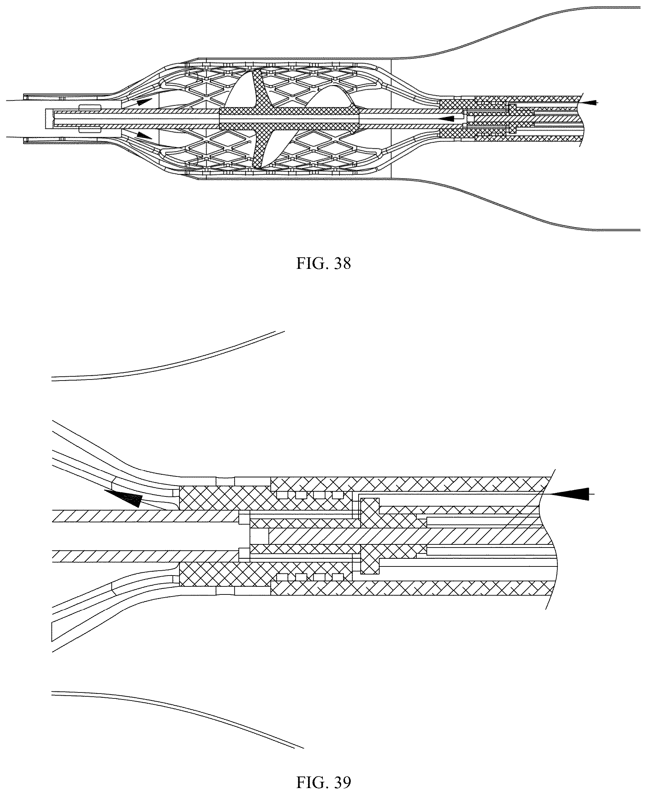

As shown in FIG. 37 to FIG. 40, this embodiment is further optimized on the basis of Embodiment 10. A sheath cover 34 is arranged at the outer side of the joint between the proximal end of the impeller rotating shaft 7 and the switching block 29. The sheath cover 34 is hermetically connected with the inner pipe 26 and the outer pipe 27 of the multi-cavity pipe 5, respectively.

In this embodiment, there is a third channel 37 between the outer wall of the proximal end of the impeller rotating shaft 7 and the inner wall of the sheath cover 34. The size of the third channel 37 is 0.003 mm-0.2 mm. The proximal end of the third channel 37 is communicated with the distal ends of the perfusion cavity 23, the first channel 30, the second channel 33 and the outflow cavity 24, respectively. The distal end of the third channel 37 is communicated with the human body.

In this embodiment, the sheath cover 34 is provided with an opening 35, so that the first channel 30, the second channel 33, the third channel 37, the perfusion cavity 23 and the outflow cavity 24 can be communicated with each other.

In this embodiment, the perfusion liquid is divided into four paths after the perfusion liquid enters from the perfusion cavity 23. The perfusion liquid in the first path enters the liquid path channel 11 through the first channel 30 and the inlet 25; meanwhile, the perfusion liquid in the second path enters the liquid path channel 11 through the second channel 33, and the perfusion liquid flows to the distal end of the impeller rotating shaft 7, flows out from the gap between the impeller rotating shaft 7 and the rotating structure 15, and then enters the human body; the perfusion fluid in the third path enters the human body through the third channel 37; and the perfusion liquid in the forth path flows out from the outflow cavity 24.

### Embodiment 12

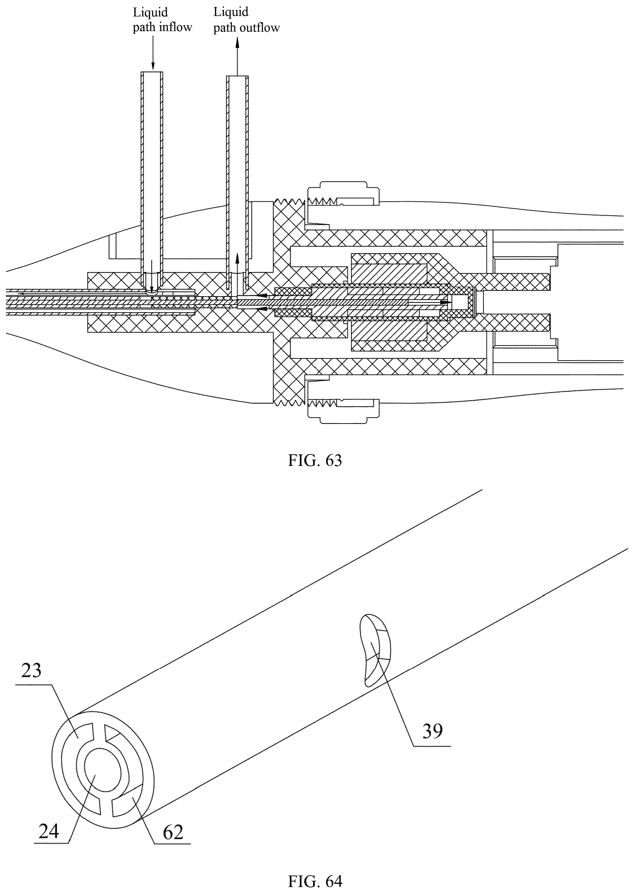

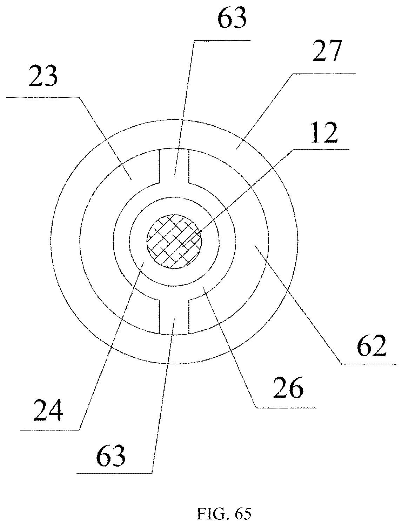

As shown in FIG. 64 to FIG. 65, the difference of this embodiment with respect to Embodiment 1 is that in this embodiment, a pressure measuring cavity 62 is arranged between the inner pipe 26 and the outer pipe 27. The pressure measuring cavity 62 is separated from the perfusion cavity 23 by a partition plate 63. The pressure measuring cavity 62 is not communicated with the perfusion cavity 23. The pressure measuring cavity 62 is communicated with the human body through the pressure measuring hole 39 formed on the side wall of the outer pipe 27. The front end of the pressure measuring hole 39 is provided with a plug 72 to prevent the pressure measuring cavity 62 from being communicated with the perfusion cavity 23 and the outflow cavity 24. A detection cavity 64 is provided in the perfusion cavity body 38. The detection cavity 64 is communicated with the pressure measuring cavity 62 and a pressure measuring pipe 65 used to pass pressure measuring liquid, respectively. A pressure measuring element 66 is arranged in the detection cavity 64. The pressure measuring element 66 is preferably a pressure sensor. The pressure measuring element 66 is configured to detect an internal pressure conducted by the pressure measuring liquid.

Since the perfusion cavity body 38 is detachably connected with the motor housing 56, the transmission line 74 of the pressure measuring element 66 is provided with a transmission line antenna 75 at a corresponding position.

### Embodiment 13

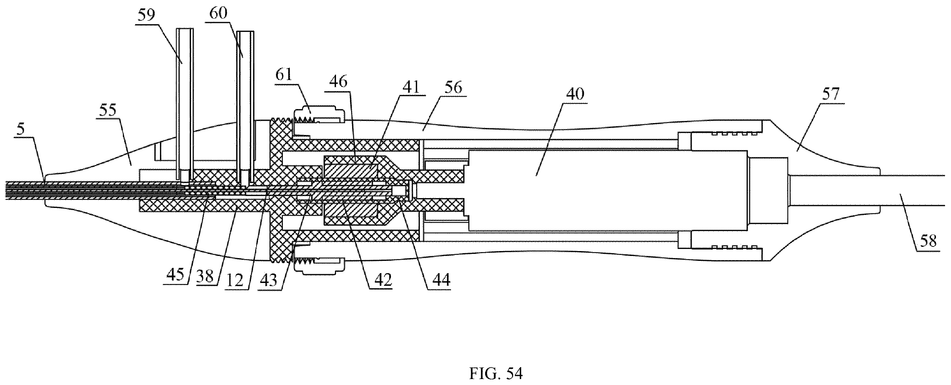

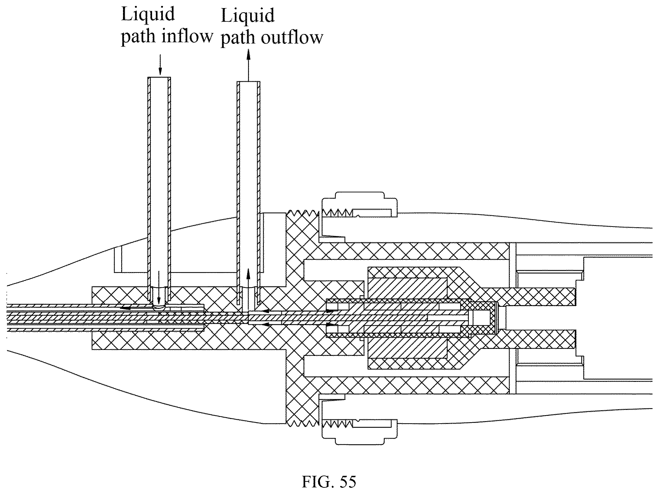

As shown in FIG. 54 to FIG. 55, the difference of this embodiment with respect to Embodiment 1 is that in this embodiment, both the internal magnetic component 42 and the external magnetic component 41 include at least two magnetic rings. Each magnetic ring in the internal magnetic component 42 is arranged in the axial direction of the driven shaft 43. Each magnetic ring in the external magnetic component 41 is arranged in the axial direction of the driven shaft 43. By increasing the volume of the internal magnetic component 42 and the external magnetic component 41 (for example, increasing the length, the thickness, the surface area, etc.), the strength of the internal magnetic component 42 and the external magnetic component 41 is increased, thereby increasing the magnetic torque.

### Embodiment 14

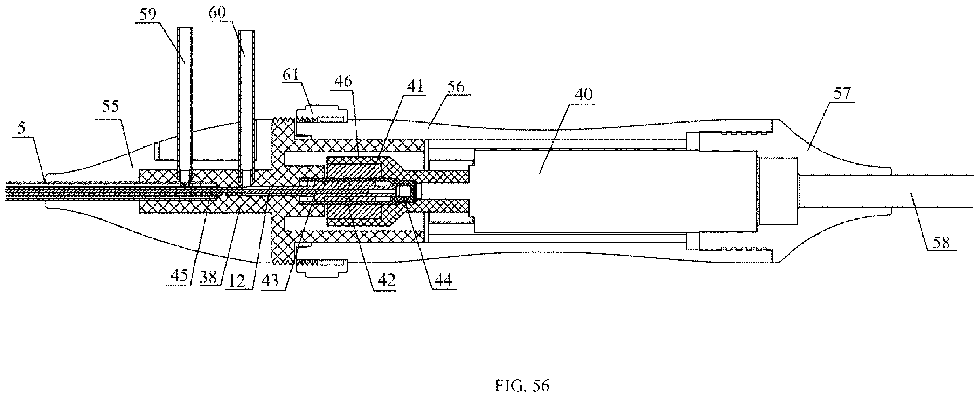

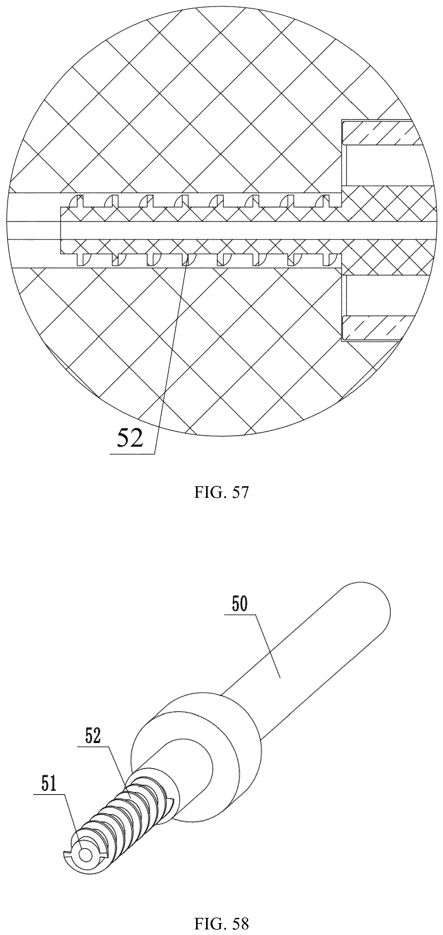

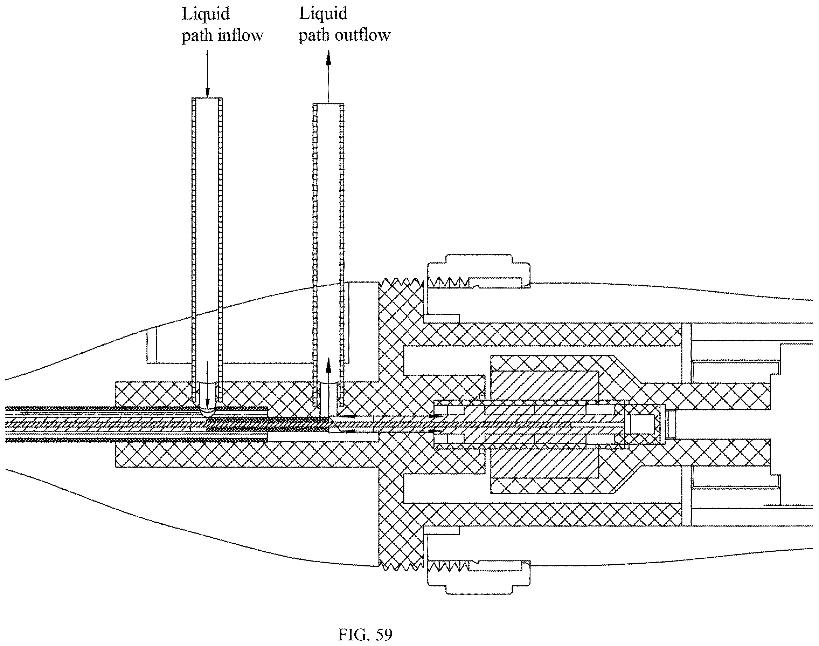

As shown in FIG. 56 to FIG. 59, the difference of this embodiment with respect to Embodiment 13 is that in this embodiment, the driven shaft 43 includes a first shaft part 50 and a second shaft part 51. The first shaft part 50 is located inside the sealing cover 44. The second shaft part 51 is located outside the sealing cover 44. A spiral structure 52 is arranged at an outer side of the second shaft part 51. A rotation direction of the spiral structure 52 is opposite to a rotation direction of the driven shaft 43. For example, if the driven shaft 43 rotates in a clockwise direction, the spiral structure 52 rotates in a counterclockwise direction. By means of providing the spiral structure 52, the liquid in the space formed by the driven shaft 43, the internal magnetic component 42 and the sealing cover 44 can be promoted to flow out, thus taking away the friction heat and the wear particles, preventing blood from existing in the space formed by the driven shaft 43, the internal magnetic component 42 and the sealing cover 44, and preventing the protein in the blood from solidifying to avoid hindering the rotation of the driven shaft 43 and the internal magnetic component 42.

### Embodiment 15

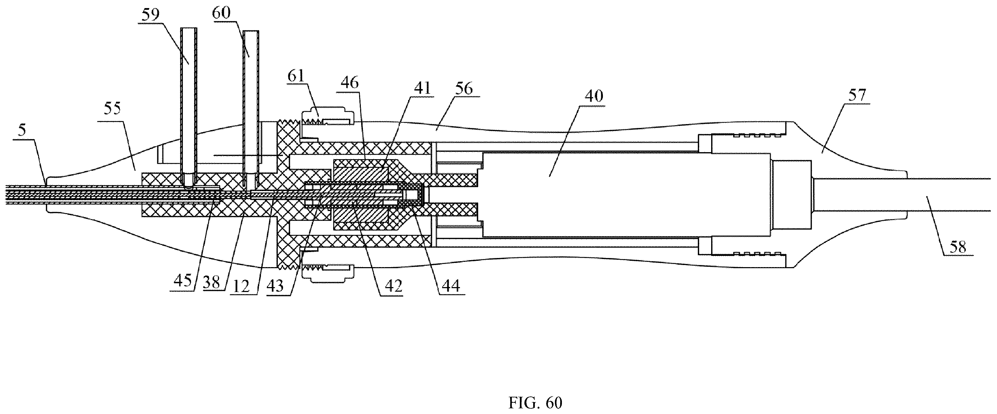

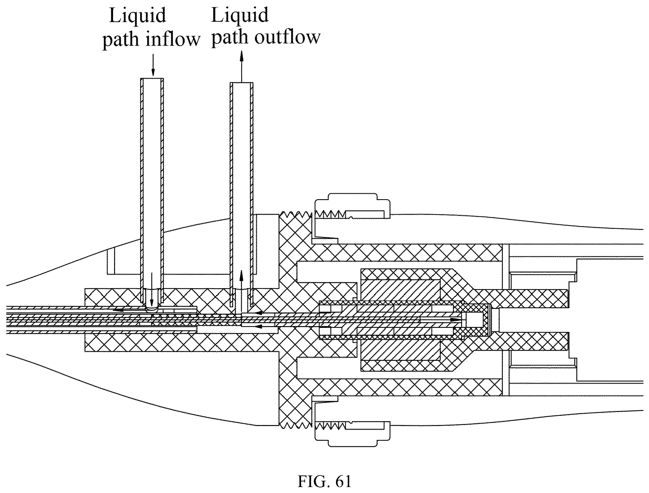

As shown in FIG. 60 to FIG. 61, the difference of this embodiment with respect to Embodiment 14 is that in this embodiment, the transmission shaft 12 is a hollow shaft. That is, a return channel is arranged inside the transmission shaft 12, and the return channel is communicated with the outflow cavity 24. Liquid is divided into two paths. Liquid in the first path flows to the space formed by the driven shaft 43, the internal magnetic component 42 and the sealing cover 44 from the gap between the driven shaft 43 and the perfusion cavity body 38, then flows out from the gap between the driven shaft 43 and the perfusion cavity body 38, thus taking away the friction heat between the driven shaft 43 and the sealing cover 44, and then flows out through the outflow pipe 60. Liquid in the second path flows into the space formed by the driven shaft 43, the internal magnetic component 42 and the sealing cover 44 through the return channel of the transmission shaft 12, and then flows out from the gap between the driven shaft 43 and the perfusion cavity body 38.

### Embodiment 16

The difference of this embodiment with respect to Embodiment 1 is that in this embodiment, both the internal magnetic component 42 and the external magnetic component 41 include at least two magnetic rings. All of the magnetic rings in the internal magnetic component 42 are sequentially nested in the radial direction of the driven shaft 43. All of the magnetic rings in the external magnetic component 41 are sequentially nested in the radial direction of the driven shaft 43.

### Embodiment 17

The difference of this embodiment with respect to Embodiment 1 is that in this embodiment, both the internal magnetic component 42 and the external magnetic component 41 include several magnetic strips uniformly arranged in the circumferential direction of the driven shaft 43. The number of magnetic strips of the internal magnetic component 42 is the same as or different from the number of the external magnetic component 41.

### Embodiment 18

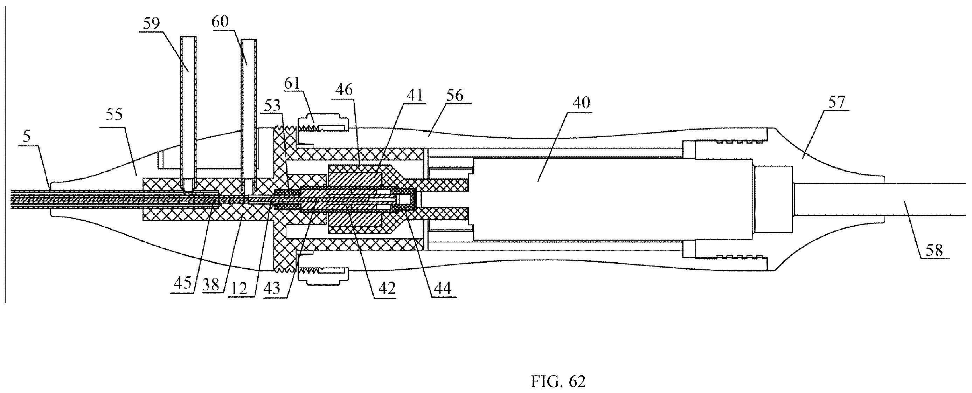

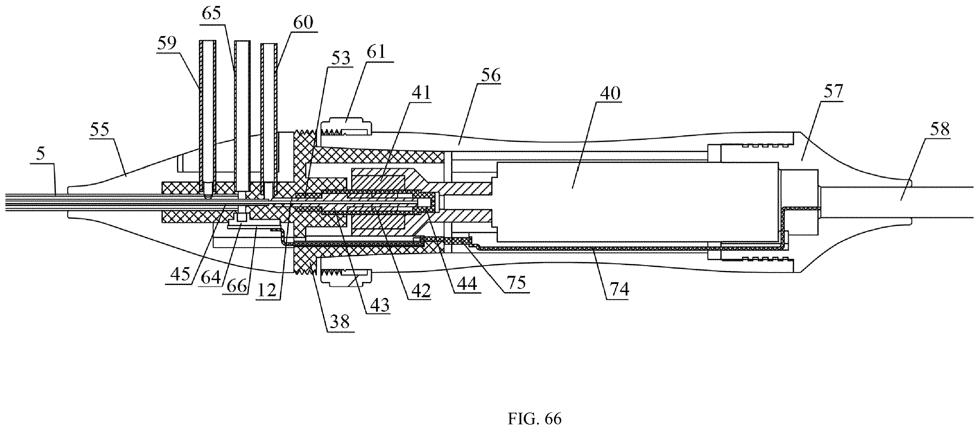

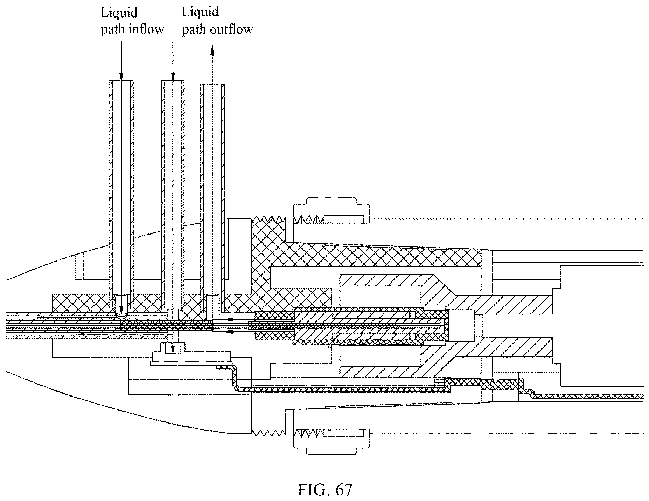

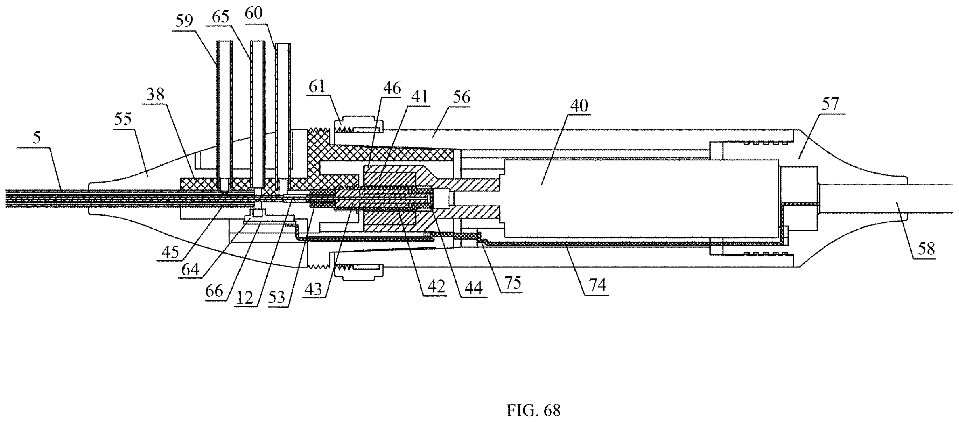

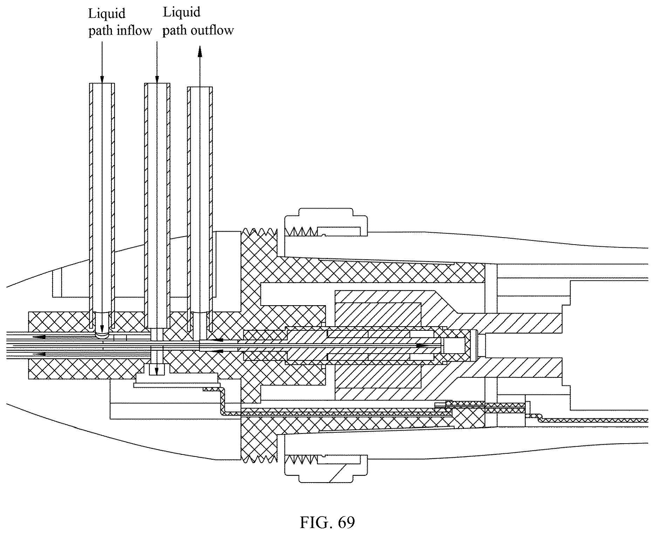

As shown in FIG. 62 to FIG. 69, the difference between this embodiment and Embodiment 12 to Embodiment 17 is that in this embodiment, a rotating shaft step 16 is not arranged on the impeller rotating shaft 7. A wear-resistant insert 53 is arranged between the driven shaft 43 and the perfusion cavity body 38. The wear-resistant insert 53 is made of ceramic materials, such as alumina and zirconia. The wear-resistant insert 53 is fixedly connected with the perfusion cavity body 38. When the driven shaft 43 rotates, the wear-resistant insert 53 forms bearing fit with the driven shaft 43 and is rotatable with respect to the driven shaft, and the wear-resistant insert 53 is matched with the projection 54 to axially limit the driven shaft 43 and restrict the driven shaft 43 from moving to the distal end of the catheter pump.

The impeller structure 2 of the present disclosure is bendable in the axial direction, which can reduce the damage of the catheter pump to the blood vessel wall in the pushing process. The impeller rotating shaft 7 is integrally injection molded with the impeller 8, and the binding force between the impeller 8 and the impeller rotating shaft 7 is increased through the connecting part on the impeller rotating shaft 7. There is a stable support in this embodiment, and the impeller rotating shaft 7 is a continuous shaft, thus avoiding the problem of a radial deflection resulting from the use of a multi-section shaft in the prior art, reducing the risk of the impeller 8 scraping with the housing 4, reducing the occurrence of hemolysis and reducing particles entering the body. There is a simple structure, low cost and high reliability in this embodiment.

### Embodiment 19

After in-depth and extensive research, it is found that most interventional instruments (such as intravascular blood pumping devices, intracardiac blood pumping devices, thrombus aspiration catheters, rotational atherectomy thrombolysis catheters, etc.) that are intervened by minimally invasive operation and transmit a torque in a human body through a flexible transmission shaft (such as a transmission shaft 12) or drive a functional unit at a distal end of a flexible transmission shaft (such as an impeller structure 2, a thrombolysis mesh basket, a rotational atherectomy blade, etc.) to rotate (such as driving the impeller 8 to rotate) are troubled by the technical problem that the functional unit and the fixed rotating shaft thereof (such as the impeller rotating shaft 7 fixed with the impeller 8 on the impeller structure 2) are worn and precipitate particles into the body/vascular blood after interacting (such as interface friction and unstable collision) with other structures (such as the pigtail pipe 1, the sheath cover 34 and the expandable bracket 3) under working conditions.

The above technical problems are particularly obvious in some interventional instruments that need to be provided with rotating shaft support components at both ends of or one end of the rotating shaft with the functions of the rotating shaft support components including a radial support and a thrust support, and these problems are difficult to solve. The rotating shaft support component includes a radial support and an axial thrust support. The rotating shaft is configured to pass through the shaft center of a functional element (such as the impeller 8) and is fixedly connected with the functional element. The radial support is configured to reduce the radial movement of the functional unit by maintaining the rotating shaft of the functional unit at a given radial position. The axial thrust support is configured to resist the force exerted on the functional unit in an axial direction and hinder the axial movement of the rotating shaft, so as to reduce or inhibit the axial position change of the functional unit. In some specific applications (such as axial-flow-type pumping blood), in addition to a radial support, the rotating shaft of the functional unit must be equipped with an axial thrust support (such as an axial-flow pump, which is different from centrifugal pump in principle, wherein its blades are subjected to the force from the fluid in the working state, that is, the fluid pushes the blades to the direction opposite to its own movement direction). In some specific applications, the axial thrust support structure of the functional unit in the interventional instrument can be arranged on the driving part outside the body (for example, in this embodiment, the impeller rotating shaft 7 is fixedly connected with the transmission shaft 12, and the proximal end of the transmission shaft 12 is fixedly connected with the driven shaft 43, so as to transfer the axial thrust support structure to the driving part outside the body) or on the rotating shaft of the functional unit inside the body (for example, in this embodiment, the distal end of the impeller rotating shaft 7 is provided with a rotating shaft step 16, and the proximal end of the rotating structure 15 is matched with the rotating shaft step 16 to axially limit the impeller rotating shaft 7 and restrict the impeller rotating shaft 7 from moving to the distal end of the catheter pump). However, when the axial thrust support structure is arranged inside the body, particles are precipitated due to the interface friction and the wear, which is not expected.

In the existing technology of liquid path design, lubrication of the axial thrust bearing interface is often neglected, so that particles precipitated due to the interface friction and the wear enter the body/vascular blood. However, in other prior art, the rotating shaft support component and the end of the rotating shaft are strengthened and hardened in terms of wear resistance.

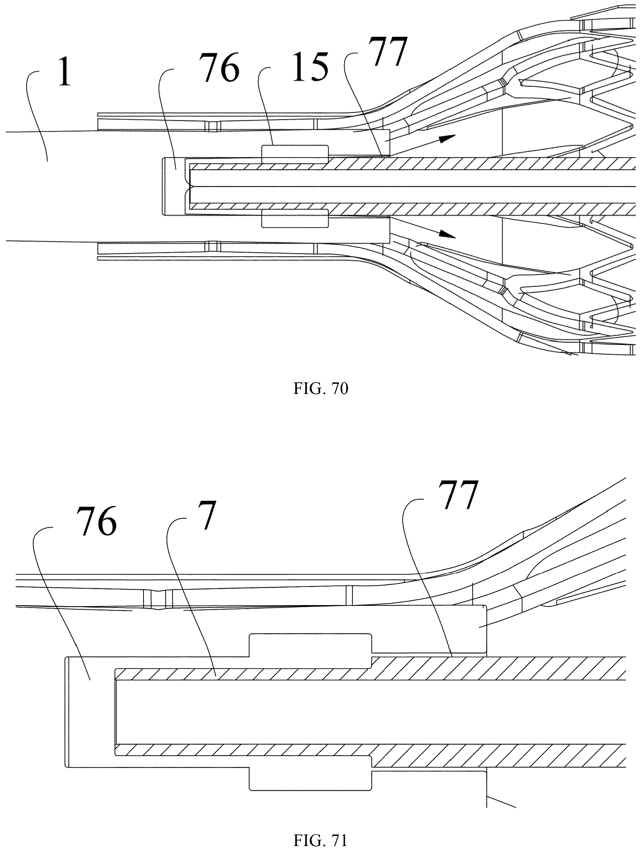

A method of inhibiting wear of a moving joint surface of a distal end section in an interventional instrument and precipitation of particles is provided in the present disclosure. The core idea of the method is that the rotating shaft is suspended by a hydraulic pressure, so that the rotating shaft is in a non-contact or near-contact state with the rotating shaft support component, so as to reduce the contact area of the moving joint surface between the rotating shaft and the rotating shaft support component. The specific method is as follows: a hydraulic chamber (that is, the rotating shaft is in gap fit with the rotating shaft support component) is arranged on the moving joint surface between the rotating shaft and the rotating shaft support component for filling liquid. The hydraulic chamber is provided with a liquid injection port and an outflow port. The liquid injection port and the outflow port are arranged in the rotating shaft, or the rotating shaft support component, or the fit gap between the rotating shaft and the rotating shaft support component. Liquid (such as physiological saline, other perfusion liquid acceptable in medicine) is injected into the hydraulic chamber through the injection port until the liquid overflows/seeps/flows out from the outflow port. The liquid pressure in the hydraulic chamber is adjusted, so that the rotating shaft is supported by the liquid in the hydraulic chamber in the radial direction and/or the axial direction and is separated from the rotating shaft support component. It can be understood that the method has been applied and practiced in Embodiment 1 of the present disclosure. That is, the perfusion liquid enters the perfusion cavity 23 through the perfusion pipe 59 and then is divided into two paths. The perfusion liquid in the first path enters the liquid path channel 11 through the inlet 25, flows to the distal end of the impeller rotating shaft 7 (as shown in FIG. 70 to FIG. 71, the hydraulic chamber 76), flows out from the gap between the impeller rotating shaft 7 and the rotating structure 15 (as shown in FIG. 70 to FIG. 71, the rotating shaft fit gap 77), and then enters the human body. When the parameters such as the injection velocity/flow rate/pressure of perfusion fluid are adjusted outside the body, the hydraulic pressure in the hydraulic chamber can be affected, so as to achieve the desired suppression of the interface friction and the wear of the distal functional unit in the interventional instrument and precipitation of particles; reduce or replace the role of the shaft sleeve or the bearing with the hydraulic support.

In some embodiments, the rotating shaft support component includes a radial support and an axial thrust support. A hydraulic chamber is arranged on the moving joint surface of the radial support and the rotating shaft, another hydraulic chamber is arranged on the moving joint surface of the axial thrust support and the rotating shaft, and the two hydraulic chambers are communicated with each other. In some other embodiments, the injection port is arranged at the end face or the side surface of the rotating shaft, and the outflow port is arranged at the fit gap between the radial support and the rotating shaft (that is, the fit gap between the radial support and the rotating shaft serves as the outflow port). In some other embodiments, the hydraulic pressure in the hydraulic chamber is greater than the hydraulic pressure at the outer side of the outflow port.

Based on the above method of inhibiting wear of the moving joint surface of the distal end section in the interventional instrument and precipitation of particles, an interventional instrument is further provided.

The interventional instrument, which includes a functional unit for rotation operation, a flexible transmission shaft, a catheter and a handle structure. The functional unit includes a rotating shaft and a functional element fixedly connected with the rotating shaft. The catheter is slender, the proximal end of the catheter is connected with the handle structure, and the distal end of the catheter is provided with a rotating shaft support structure which can provide a radial support and an axial thrust support to the rotating shaft. The flexible transmission shaft is arranged in and passes through the conduit, the distal end of the flexible transmission shaft is connected with the rotating shaft, and the proximal end of the flexible transmission shaft is connected with the handle structure to transmit the torque output by the handle structure to the rotating shaft. The functional unit can perform limited axial displacement with respect to the rotating shaft support structure. A hydraulic chamber is arranged on the moving joint surface between the rotating shaft support structure and the rotating shaft, and the hydraulic chamber is configured to enable the rotating shaft supported by liquid in the radial direction and/or the axial direction to be separated from the moving joint surface of the rotating shaft support structure. The hydraulic chamber is provided with a liquid injection port and an outflow port.

In some embodiments, the functional unit of the interventional instrument is one of an axial-flow-type impeller structure, a thrombolysis mesh basket structure and a rotational atherectomy structure.

In some embodiments, the interventional instrument further includes a perfusion liquid path. The liquid inlet and the liquid outlet of the perfusion liquid path are arranged at the proximal end section of the catheter close to the handle structure. The rotating shaft is configured as a hollow channel or is provided with an axial hole channel. The channel or the hole channel of the rotating shaft enables the perfusion liquid path to be communicated with the injection port of the hydraulic chamber.

In some embodiments, the catheter of the interventional instrument is a multi-cavity pipe. The flexible transmission shaft and the perfusion liquid path are arranged in different cavity channels in the catheter, respectively.

In some embodiments, the interventional instrument further includes an outer cover structure (such as an expandable bracket 3) for supporting and maintaining the cavity for the functional unit. The rotating shaft support structure is configured at the distal end section of the rotating shaft. The distal end section of the outer cover structure is fixedly connected with the rotating shaft support structure, and the proximal end section of the outer cover structure is fixedly connected with the distal end section of the catheter.

In some embodiments, the interventional instrument further includes a distal end support structure (such as a pigtail pipe 1). The rotating shaft support structure are integrally arranged with the distal end support structure. The distal end section of the outer cover structure is fixedly connected with the proximal end section of the distal end support structure. The proximal end section of the outer cover structure is fixedly connected with the distal end section of the catheter.

In some embodiments, the interventional instrument is configured to pump blood in the heart or aspirate thrombus in the blood vessel cavity.

### Embodiment 20

After in-depth and extensive research, it is also found that for most interventional instruments that need to rotate through the rotating shaft, the over-bending performance of the rotating shaft (that is, the rotating shaft can adapt to the bending radius of different turns when passing through the tortuous vascular structure) and the rotational stability (that is, the rotating shaft can keep the axis from oscillating and swinging under the rotating operation of the load functional element) are a pair of performances which are difficult to be taken into account at the same time. Meanwhile, the firmness of the connection between the rotating shaft and the functional component is also one of the safety factors that interventional instruments mainly take into account. The excellent over-bending performance requires the rotating shaft to have good flexible bending deformation ability, and the excellent rotational stability requires the rotating shaft to have good ability of bending moment deformation resistance (that is, the rigidity of the shaft lever). In the prior art, such as a heart blood pumping device or a thrombus aspiration device, the length of the rotating shaft is often extremely compressed and shortened in order to take both over-bending performance and the rotational stability into account, abandon the double-end/side radial support/limit, and select the single-end/side radial support/limit. This leads to the following technical problems: the connection firmness is obviously reduced due to the reduction of the connection joint surface between the functional element and the rotating shaft, and the anti-interference performance of the whole functional unit is reduced. Therefore, a method of strengthening a rotating shaft of an interventional instrument is provided in the present disclosure. The core idea of the method is as follows: two materials with different properties are used for embedded configuration with complementary elasticity and rigidity, so that the rotating shaft can be balanced and improved in the aspects of over-bending performance and the rotational stability which are taken into account at the same time. Meanwhile, the connection firmness between the rotating shaft and the functional element can be improved. For example, an elastic polymer material is embedded in the direction that the outer surface of the rotating shaft with a metal body faces the shaft center (the axis) (it can also be understood that a part of the body of the metal rotating shaft is replaced by elastic polymer materials from the outer surface to the axis). The specific method includes the following steps: several grooves or through holes are etched from the outer side of the rotating shaft to the axial direction on a rotating shaft with a body having two end faces and an outer side surface made of metal; grooves or through holes are arranged in an array in the axial direction and the circumferential direction of the rotating shaft to change the deflection of the metal rotating shaft; the grooves or through holes arranged towards these arrays on the outer side of the rotating shaft are filled with elastic polymer materials and the elastic polymer materials are adhered to the wall surfaces of the grooves or through holes. It can be understood that the method has been applied and practiced in Embodiment 1 to Embodiment 9 of the present disclosure. That is, the groove 17 or the through hole 18 of the impeller rotating shaft 7 on the impeller structure 2 is filled with the hub 9. For the metal rotating shaft with changed deflection and embedded with elastic polymer materials, under the rotating operation, the elastic polymer material in the groove or the through hole can elastically pull and elastically compress the adhered metal wall surface, so that the deflection and oscillation of the rotating shaft off the axis can be suppressed, and the rotating shaft can be quickly corrected in terms of deflection to maintain its rotational stability after being interfered by the bending moment.

In some embodiments, the elastic polymer material filling each groove or through hole overflows the metal outer side surface of the metal rotating shaft and is piled up to form an elastic polymer coating layer (such as the hub 9) with a continuous outer side surface.

In some embodiments, the elastic polymer coating layer is molded into a three-dimensional contour structure (such as the blade 10) with the function of a pump blade.

In some embodiments, the metal rotating shaft is configured as a hollow metal pipe rotating shaft. The pipe wall of the hollow metal pipe rotating shaft is etched to form a crack penetrating through the inner and outer wall surfaces of the pipe wall. The crack is filled with elastic polymer materials, which overflows the crack to form an elastic polymer coating layer of a continuous surface (such as a film layer 19 and a hub 9) on the inner and outer wall surfaces of the pipe wall. The elastic polymer coating layer is adhered with the wall surface of the metal pipe. It can be understood that: in Embodiment 1, the integrated injection molding process is only one of the means to achieve the combination of the metal rotating shaft and the elastic polymer material.

In some embodiments, the hollowed-out cracks of the metal pipe rotating shaft are arranged at intervals in the axial direction of the metal pipe rotating shaft and staggered in the circumferential direction of the metal pipe rotating shaft. Alternatively, the hollowed-out cracks extend spirally along the central axis of the metal pipe rotating shaft.

In some embodiments, in the axial direction of the metal pipe rotating shaft, only the middle section of the metal pipe rotating shaft is etched and hollowed out with cracks, while the two end sections of the metal pipe rotating shaft have no hollowed-out cracks.

The "rotating shaft", "metal rotating shaft" and "metal pipe rotating shaft" mentioned in this embodiment all refer to the shafts used to fixedly connect and support functional elements (such as an impeller, a blade, a mesh basket, and a rotational atherectomy blade) and transmit a torque to these functional elements. The shaft can be an independent shaft from the flexible transmission shaft (used to drive the source or handle structure to transmit a torque to the functional unit, and referred to as "flexible transmission shaft" in this embodiment) or a shaft integrated with the flexible transmission shaft.

Based on the method of strengthening a rotating shaft of an interventional instrument provided in the present disclosure, a functional unit of an interventional instrument is further provided.

The functional unit of an interventional instrument, wherein the functional unit includes a functional element for rotation operation and a metal rotating shaft for supporting the functional element and transmitting a torque to the functional element. The functional element includes a body and a base which are integrated with each other. The outer side surface of the metal rotating shaft is provided with grooves or through holes in the circumferential direction, and the grooves or through holes are configured for changing the bending deflection of the metal rotating shaft. The grooves or through holes are arranged in an array in the axial direction of the metal rotating shaft or spirally arranged in the axial direction of the metal rotating shaft. The base of the functional element is made of elastic polymer materials. A part of the base of the functional element is embedded in the groove or through hole of the metal rotating shaft and adhered to the wall surface of the groove or through hole. The functional element is fixedly connected with the metal rotating shaft through the base; and the body of the functional element is a three-dimensional contour structure with the function of a pump blade.

In some embodiments, the metal rotating shaft is a hollow metal pipe rotating shaft.

In some embodiments, a part of the functional element base passes through the through hole of the metal pipe rotating shaft, and a continuous layered structure is formed on the inner wall surface of the metal pipe rotating shaft.

In some embodiments, the body of the functional element is a blade, and the base of the functional element is a hub.

In some embodiment, the metal pipe rotating shaft includes a first rigid section, a flexible section and a second rigid section which are sequentially arranged, the through hole is located in the flexible section, and the flexible section is bendable.

### Embodiment 21

A blood pumping device includes a functional unit for pumping blood, a flexible transmission shaft, a catheter and a handle structure. The functional unit includes a metal rotating shaft and an impeller. The metal rotating shaft is configured for supporting the impeller and transmitting a torque to the impeller. The catheter is slender, the proximal end of the catheter is connected with the handle structure, and the distal end of the catheter is provided with a rotating shaft support structure which can provide a radial support to the metal rotating shaft. The flexible transmission shaft is arranged in and passes through the conduit, the distal end of the flexible transmission shaft is connected with the metal rotating shaft, the proximal end of the flexible transmission shaft is connected with the handle structure to transmit the torque output by the handle structure to the metal rotating shaft. The functional unit can perform limited axial displacement with respect to the rotating shaft support structure. The impeller includes a hub and a blade. The outer side surface of the metal rotating shaft is provided with grooves or through holes in the circumferential direction, and the grooves or through holes are configured for changing the bending deflection of the metal rotating shaft. The grooves or through holes are arranged in an array in the axial direction of the metal rotating shaft or spirally arranged in the axial direction of the metal rotating shaft. The hub is made of elastic polymer materials. A part of the hub is embedded in the groove or through hole of the metal rotating shaft and adhered to the wall surface of the groove or through hole. The impeller is fixedly connected with the metal rotating shaft through the hub.

In some embodiments, a part of the hub passes through the through hole of the metal pipe rotating shaft, and a continuous layered structure is formed on the inner wall surface of the metal pipe rotating shaft.

The metal pipe rotating shaft includes a first rigid section, a flexible section and a second rigid section which are sequentially arranged, the through hole is located in the flexible section, and the flexible section is bendable.

In some embodiments, the rotating shaft support structure at the distal end of the catheter further has an axial thrust support function. The first rigid section or the second rigid section of the metal pipe rotating shaft is arranged in the rotating shaft support structure with an axial thrust support.

In some embodiments, a hydraulic chamber is arranged between the rotating shaft support structure and the moving joint surface of the first rigid section or the second rigid section, and the hydraulic chamber is configured to enable the rotating shaft supported by liquid in the radial direction and/or the axial direction to be separated from the moving joint surface of the rotating shaft support structure. The hydraulic chamber is provided with a liquid injection port and an outflow port.

In some embodiments, the blood pumping device further includes a perfusion liquid path. The liquid inlet and the liquid outlet of the perfusion liquid path are arranged at the proximal end section of the catheter close to the handle structure. The metal rotating shaft is configured as a hollow channel or is provided with an axial hole channel. The channel or the hole channel of the metal rotating shaft enables the perfusion liquid path to be communicated with the injection port of the hydraulic chamber.

In some embodiments, the catheter of the blood pumping device is a multi-cavity pipe. The flexible transmission shaft and the perfusion liquid path are arranged in different cavity channels in the catheter.

In some embodiments, the blood pumping device further includes an outer cover structure (such as an expandable bracket 3) for supporting and maintaining the cavity for the functional unit. The rotating shaft support structure is configured at the distal end section of the metal rotating shaft. The distal end section of the outer cover structure is fixedly connected with the rotating shaft support structure, and the proximal end section of the outer cover structure is fixedly connected with the distal end section of the catheter.

In some embodiments, the blood pumping device further includes a distal end support structure (such as a pigtail pipe 1). The rotating shaft support structure is integrally arranged with the distal end support structure. The distal end section of the outer cover structure is fixedly connected with the proximal end section of the distal end support structure. The proximal end section of the outer cover structure is fixedly connected with the distal end section of the catheter.

In the present disclosure, the proximal end refers to the end near the doctor, and the distal end refers to the end far away from the doctor.

In this specification, specific examples are used to explain the principle and implementation of the present disclosure. The description of the above embodiments is only used to help understand the method and the core idea of the present disclosure. In addition, for those skilled in the art, modifications and changes in terms of the specific implementation and the application scope according to the idea of the present disclosure can be made. In summary, the contents of this specification should not be construed as limiting the present disclosure.

## Claims

1. A catheter pump, comprising a functional module, wherein the functional module comprises an impeller structure (2), the impeller structure (2) comprises an impeller rotating shaft (7) and an impeller (8), the impeller (8) comprises a hub (9) and several blades (10), the several blades (10) are arranged on the hub (9), a side wall of the impeller rotating shaft (7) is provided with a connecting part (17, 18), the hub (9) is connected with the connecting part (17, 18) by integral injection molding, and the connecting part (17, 18) is configured to limit relative movement of the impeller (8);
wherein the connecting part is a groove (17) and/or a through hole (18);
**characterized in that**
the impeller rotating shaft (7) comprises a first rigid section (20), a flexible section (21) and a second rigid section (22) which are sequentially arranged, the connecting part (17, 18) is located in the flexible section (21), and the flexible section (21) is bendable.

2. The catheter pump according to claim 1, further comprising a pigtail pipe (1), an expandable bracket (3), a housing (4), a multi-cavity pipe (5) and a handle module (6), wherein the impeller rotating shaft (7) is rotatably connected with the pigtail pipe (1), the impeller rotating shaft (7) is connected with a mechanical power source in the handle module (6) through a transmission shaft (12) in the multi-cavity pipe (5), the expandable bracket (3) is located at an outer side of the impeller structure (2), a distal end of the expandable bracket (3) is connected with the pigtail pipe (1), a proximal end of the expandable bracket (3) is connected with the multi-cavity pipe (5), the housing (4) is located at an outer side of the expandable bracket (3), the housing (4) is connected with the pigtail pipe (1) and the multi-cavity pipe (5), respectively, and the housing (4) is provided with a blood inlet window (13) and a blood outlet window (14).

3. The catheter pump according to claim 2, wherein a distal end of the impeller rotating shaft (7) is provided with a rotating shaft step (16), the impeller rotating shaft (7) is rotatably connected with the pigtail pipe (1) through a rotating structure (15), and a proximal end of the rotating structure (15) is matched with the rotating shaft step (16) to axially limit the impeller rotating shaft (7) and restrict the impeller rotating shaft (7) from moving to a distal end of the catheter pump.

4. The catheter pump according to claim 1, comprising a liquid path module, a rotating structure (15) is provided between a distal end of the impeller rotating shaft (7) and a pigtail pipe (1), ; the liquid path module comprises a multi-cavity pipe (5), the multi-cavity pipe (5) comprises a perfusion cavity (23); perfusion liquid flows into the distal end of the impeller rotating shaft (7) from the perfusion cavity (23), flows out from a gap between the impeller rotating shaft (7) and the rotating structure (15), and then enters a human body.

5. The catheter pump according to claim 4, further comprising an expandable bracket (3), a housing (4) and a handle module (6), wherein the impeller rotating shaft (7) is connected with a mechanical power source in the handle module (6) through a transmission shaft (12) in the multi-cavity pipe (5), the expandable bracket (3) is located at an outer side of the impeller structure (2), a distal end of the expandable bracket (3) is connected with the pigtail pipe (1), a proximal end of the expandable bracket (3) is connected with the multi-cavity pipe (5), the housing (4) is located at an outer side of the expandable bracket (3), the housing (4) is connected with the pigtail pipe (1) and the multi-cavity pipe (5), respectively, and the housing (4) is provided with a blood inlet window (13) and a blood outlet window (14).

6. The catheter pump according to claim 5, wherein the multi-cavity pipe (5) is further provided with an outflow cavity (24), the perfusion cavity (23) extends from one end of the multi-cavity pipe (5) to another end of the multi-cavity pipe (5), the outflow cavity (24) extends from one end of the multi-cavity pipe (5) to another end of the multi-cavity pipe (5), a proximal end of the impeller rotating shaft (7) is connected with a distal end of the transmission shaft (12), a liquid path channel (11) is arranged in the impeller rotating shaft (7), the impeller rotating shaft (7) is provided with an inlet (25) communicated with the liquid path channel (11), the inlet (25) is communicated with the perfusion cavity (23), the liquid path channel (11) and the outflow cavity (24), respectively; perfusion liquid flows from the perfusion cavity (23), flows into the liquid path channel (11) from the proximal end of the impeller rotating shaft (7) through the inlet (25), flows along the liquid path channel (11) to the distal end of the impeller rotating shaft (7), flows out from the gap between the impeller rotating shaft (7) and the rotating structure (15), and then enters the human body, and the perfusion liquid flows out from the outflow cavity (24).

7. The catheter pump according to claim 6, wherein the multi-cavity pipe (5) comprises an inner pipe (26) and an outer pipe (27), the inner pipe (26) is coaxially arranged at an inner side of the outer pipe (27), the perfusion cavity (23) is arranged between the inner pipe (26) and the outer pipe (27), the transmission shaft (12) is located in the inner pipe (26), and the outflow cavity (24) is formed between the transmission shaft (12) and the inner pipe (26).

8. The catheter pump according to claim 7, wherein a spring pipe (28) is arranged between the transmission shaft (12) and the inner pipe (26).

9. The catheter pump according to claim 6, wherein the proximal end of the impeller rotating shaft (7) is connected with the distal end of the transmission shaft (12) through a switching block (29), the switching block (29) is provided with a first channel (30), and the first channel (30) is communicated with the inlet (25), the perfusion cavity (23) and the outflow cavity (24), respectively.

10. The catheter pump according to claim 9, wherein the proximal end of the impeller rotating shaft (7) is provided with protrusions (31), the inlet (25) is formed between the adjacent protrusions (31), the switching block (29) is provided with a chute (32), the protrusion (31) is in sliding connection with the chute (32), a second channel (33) is formed between an inner surface of the protrusion (31) and a surface of the chute (32), and the second channel (33) is communicated with the perfusion cavity (23) and the liquid path channel (11), respectively.

11. The catheter pump according to claim 10, wherein the second channel (33) is further communicated with the first channel (30) and the outflow cavity (24).

12. The catheter pump according to claim 9, wherein a sheath cover (34) is arranged at an outer side of a joint between the proximal end of the impeller rotating shaft (7) and the switching block (29), the sheath cover (34) is connected with the expandable bracket (3) and the multi-cavity pipe (5), respectively, an opening (35) is formed on the sheath cover (34), and a distal end of the perfusion cavity (23) is communicated with a distal end of the outflow cavity (24) through the opening (35).

13. The catheter pump according to claim 12, wherein the sheath cover (34) is provided with a sheath cover limiting step (36), the sheath cover limiting step (36) is matched with the proximal end of the impeller rotating shaft (7) and the proximal end of the switching block (29) to axially limit the impeller rotating shaft (7) and restrict the impeller rotating shaft (7) from moving to the proximal end of the catheter pump.

14. The catheter pump according to claim 12, wherein a third channel (37) is arranged between the proximal end of the impeller rotating shaft (7) and the sheath cover (34), a proximal end of the third channel (37) is communicated with the perfusion cavity (23), and the distal end of the third channel (37) is communicated with the human body.

15. The catheter pump according to claim 14, wherein the third channel (37) is further communicated with the first channel (30) and the outflow cavity (24).

16. The catheter pump according to claim 1 comprising a handle module (6), wherein the handle module (6) comprises a transmission sealing structure, the transmission sealing structure comprises an external magnetic component (41), an internal magnetic component (42), a driven shaft (43) and a sealing cover (44), the internal magnetic component (42) is arranged on the driven shaft (43), the driven shaft (43) is connected with the impeller rotating shaft (7) through a transmission shaft (12), the external magnetic component (41) is arranged at an outer side of the internal magnetic component (42), the external magnetic component (41) is connected with a mechanical power source, the sealing cover (44) is configured to isolate the internal magnetic component (42) from the external magnetic component (41); and the driven shaft (43) rotates with respect to the sealing cover (44) and moves in an axial direction.

17. The catheter pump according to claim 16, further comprising a pigtail pipe (1), an expandable bracket (3), a housing (4) and a liquid path module, wherein the impeller rotating shaft (7) is rotatably connected with the pigtail pipe (1) through a rotating structure (15), the liquid path module comprises a multi-cavity pipe (5), the transmission shaft (12) is located in the multi-cavity pipe (5), the expandable bracket (3) is located at an outer side of the impeller structure (2), a distal end of the expandable bracket (3) is connected with the pigtail pipe (1), a proximal end of the expandable bracket (3) is connected with the multi-cavity pipe (5), the housing (4) is located at an outer side of the expandable bracket (3), the housing (4) is connected with the pigtail pipe (1) and the multi-cavity pipe (5), respectively, and the housing (4) is provided with a blood inlet window (13) and a blood outlet window (14).

18. The catheter pump according to claim 17, wherein the handle module (6) further comprises a perfusion cavity body (38), a perfusion cavity (23) of the multi-cavity pipe (5) is communicated with a perfusion port of the perfusion cavity body (38), an outflow cavity (24) of the multi-cavity pipe (5) is communicated with an outflow port of the perfusion cavity body (38), a proximal end of the multi-cavity pipe (5) is connected with the perfusion cavity body (38), a proximal end of the perfusion cavity (23) is not communicated with a proximal end of the outflow cavity (24), and the sealing cover (44) is connected with the perfusion cavity body (38).

19. The catheter pump according to claim 18, wherein the internal magnetic component (42) is sleeved on the driven shaft (43) and is fixedly connected with the driven shaft (43), the sealing cover (44) is arranged at an outer side of the internal magnetic component (42), the external magnetic component (41) is arranged at an outer side of the sealing cover (44), an opening end (47) of the sealing cover (44) is hermetically connected with the perfusion cavity body (38), and both the driven shaft (43) and the internal magnetic component (42) are capable of moving in an axial direction of the sealing cover (44).

20. The catheter pump according to claim 19, wherein one end of the driven shaft (43) protrudes from the opening end (47) of the sealing cover (44), a gap is formed between the driven shaft (43) and the perfusion cavity body (38), the gap between the driven shaft (43) and the perfusion cavity body (38) is communicated with the outflow cavity (24), an other end of the driven shaft (43) is close to a sealing end (48) of the sealing cover (44), a proximal end of the impeller rotating shaft (7) is fixedly connected with a distal end of the transmission shaft (12), the movement of the driven shaft (43) is limited by the sealing end (48), and gaps are formed between the driven shaft (43) and the sealing cover (44), and between the internal magnetic component (42) and the sealing cover (44), respectively.

21. The catheter pump according to claim 16, wherein the driven shaft (43) has a solid structure, and one end of the driven shaft (43) is connected with the transmission shaft (12).

22. The catheter pump according to claim 16, wherein the driven shaft (43) has a hollow structure, and the transmission shaft (12) extends into the driven shaft (43).

23. The catheter pump according to claim 18, wherein the transmission shaft (12) is a hollow shaft, a return channel is arranged inside the transmission shaft (12), and the return channel is communicated with the outflow cavity (24).

24. The catheter pump according to claim 16, wherein the driven shaft (43) is an optical axis.

25. The catheter pump according to claim 16, wherein the driven shaft (43) comprises a first shaft part (50) and a second shaft part (51), the first shaft part (50) is located inside the sealing cover (44), the second shaft part (51) is located outside the sealing cover (44), a spiral structure (52) is arranged at an outer side of the second shaft part (51), and a rotation direction of the spiral structure (52) is opposite to a rotation direction of the driven shaft (43).

26. The catheter pump according to claim 18, wherein a wear-resistant insert (53) is arranged between the driven shaft (43) and the perfusion cavity body (38), the wear-resistant insert (53) is fixedly connected with the perfusion cavity body (38), the wear-resistant insert (53) is rotatable with respect to the driven shaft (43), the driven shaft (43) is provided with a projection (54), and the wear-resistant insert (53) is matched with the projection (54) to axially limit the driven shaft (43) and restrict the driven shaft (43) from moving to a distal end of the catheter pump.

27. The catheter pump according to claim 18, wherein the handle module (6) further comprises a first diffusion stress pipe (55), a motor housing (56), a second diffusion stress pipe (57) and a cable (58), one end of the first diffusion stress pipe (55) is connected with the multi-cavity pipe (5), an other end of the first diffusion stress pipe (55) is connected with the perfusion cavity body (38), the first diffusion stress pipe (55) is provided with a perfusion pipe (59) and an outflow pipe (60), one end of the perfusion pipe (59) is communicated with the perfusion port, one end of the outflow pipe (60) is communicated with the outflow port, the motor housing (56) is arranged at an outer side of a driving motor (40), the motor housing (56) is detachably connected with the perfusion cavity body (38), the motor housing (56) is connected with the perfusion cavity body (38) through a locking nut (61), one end of the second diffusion stress pipe (57) is connected with the motor housing (56), an other end of the second diffusion stress pipe (57) is connected with the cable (58), and one end of the cable (58) is connected with the driving motor (40).

28. The catheter pump according to claim 18, wherein the multi-cavity pipe (5) comprises an inner pipe (26) and an outer pipe (27), the inner pipe (26) is coaxially arranged at an inner side of the outer pipe (27), the perfusion cavity (23) and a pressure measuring cavity (62) are arranged between the inner pipe (26) and the outer pipe (27), the pressure measuring cavity (62) is separated from the perfusion cavity (23) by a partition plate (63), the pressure measuring cavity (62) is not communicated with the perfusion cavity (23), the transmission shaft (12) is located in the inner pipe (26), the outflow cavity (24) is provided between the transmission shaft (12) and the inner pipe (26), the pressure measuring cavity (62) is communicated with a human body through a pressure measuring hole (39) formed on a side wall of the outer pipe (27), a detection cavity (64) is arranged in the perfusion cavity body (38), the detection cavity (64) is respectively communicated with the pressure measuring cavity (62) and a pressure measuring pipe (65) which is configured to allow pressure measuring liquid to pass through, a pressure measuring element (66) is arranged in the detection cavity (64), and the pressure measuring element (66) is configured to detect an internal pressure conducted by the pressure measuring liquid.

29. The catheter pump according to claim 1wherein a liquid path channel (11) is arranged in the impeller rotating shaft (7), the connecting part is the through hole (18); the liquid path channel (11) is communicated with the through hole (18), and the hub (9) passes through the through hole (18) and forms a film layer (19) on an inner wall of the impeller rotating shaft (7).

30. The catheter pump according to claim 1, wherein the flexible section (21) has a hypotube structure.

## Patentansprüche

1. Katheterpumpe, umfassend ein Funktionsmodul, wobei das Funktionsmodul eine Laufradstruktur (2) umfasst, die Laufradstruktur (2) eine Laufrad-Drehwelle (7) und ein Laufrad (8) umfasst, das Laufrad (8) eine Nabe (9) und mehrere Schaufeln (10) umfasst, die mehreren Schaufeln (10) an der Nabe (9) angeordnet sind, eine Seitenwand der Laufrad-Drehwelle (7) mit einem Verbindungsteil (17, 18) versehen ist, die Nabe (9) durch integrales Spritzgießen mit dem Verbindungsteil (17, 18) verbunden ist und das Verbindungsteil (17, 18) so ausgebildet ist, dass es eine Relativbewegung des Laufrads (8) begrenzt; wobei das Verbindungsteil eine Nut (17) und/oder eine Durchgangsbohrung (18) ist; **dadurch gekennzeichnet, dass** die Laufrad-Drehwelle (7) einen ersten starren Abschnitt (20), einen flexiblen Abschnitt (21) und einen zweiten starren Abschnitt (22) umfasst, die nacheinander angeordnet sind, sich das Verbindungsteil (17, 18) im flexiblen Abschnitt (21) befindet und der flexible Abschnitt (21) biegbar ist.

2. Katheterpumpe nach Anspruch 1, ferner umfassend ein Pigtail-Rohr (1), eine ausdehnbare Klammer (3), ein Gehäuse (4), ein Mehrkammerrohr (5) und ein Griffmodul (6), wobei die Laufrad-Drehwelle (7) drehbar mit dem Pigtail-Rohr (1) verbunden ist, die Laufrad-Drehwelle (7) mit einer Übertragungswelle (12) im Mehrkammerrohr (5) mit einer mechanischen Antriebsquelle im Griffmodul (6) verbunden ist, die ausdehnbare Klammer (3) an einer Außenseite der Laufradstruktur (2) angeordnet ist, ein distales Ende der ausdehnbaren Klammer (3) mit dem Pigtail-Rohr (1) verbunden ist, ein proximales Ende der ausdehnbaren Klammer (3) mit dem Mehrkammerrohr (5) verbunden ist, das Gehäuse (4) an einer Außenseite der ausdehnbaren Klammer (3) angeordnet ist, das Gehäuse (4) jeweils mit dem Pigtail-Rohr (1) und dem Mehrkammerrohr (5) verbunden ist, und das Gehäuse (4) mit einem Bluteinlassfenster (13) und einem Blutauslassfenster (14) versehen ist.

3. Katheterpumpe nach Anspruch 2, wobei ein distales Ende der Laufrad-Drehwelle (7) mit einer Drehwellenstufe (16) versehen ist, die Laufrad-Drehwelle (7) mittels einer Drehvorrichtung (15) drehbar mit dem Pigtail-Rohr (1) verbunden ist, und ein proximales Ende der Drehvorrichtung (15) mit der Drehwellenstufe (16) in Eingriff steht, um die Laufrad-Drehwelle (7) axial zu begrenzen und zu verhindern, dass sich die Laufrad-Drehwelle (7) in Richtung eines distalen Endes der Katheterpumpe bewegt.

4. Katheterpumpe nach Anspruch 1, umfassend ein Flüssigkeitswegmodul, wobei eine Drehvorrichtung (15) zwischen einem distalen Ende der Laufrad-Drehwelle (7) und einem Pigtail-Rohr (1) vorgesehen ist, wobei das Flüssigkeitswegmodul ein Mehrkammerrohr (5) umfasst, wobei das Mehrkammerrohr (5) eine Perfusionskammer (23) umfasst; Perfusionsflüssigkeit aus der Perfusionskammer (23) in das distale Ende der Laufrad-Drehwelle (7) fließt, aus einem Spalt zwischen der Laufrad-Drehwelle (7) und der rotierenden Struktur (15) austritt und dann in den menschlichen Körper gelangt.

5. Katheterpumpe nach Anspruch 4, die ferner eine expandierbare Klammer (3), ein Gehäuse (4) und ein Griffmodul (6) umfasst, wobei die Laufrad-Drehwelle (7) mittels einer Übertragungswelle (12) im Mehrkammerrohr (5) mit einer mechanischen Antriebsquelle im Griffmodul (6) verbunden ist, die ausdehnbare Klammer (3) an einer Außenseite der Laufradstruktur (2) angeordnet ist, ein distales Ende der ausdehnbaren Klammer (3) mit dem Pigtail-Rohr (1) verbunden ist, ein proximales Ende der ausdehnbaren Klammer (3) mit dem Mehrkammerrohr (5) verbunden ist, das Gehäuse (4) an einer Außenseite der ausdehnbaren Klammer (3) angeordnet ist, das Gehäuse (4) jeweils mit dem Pigtail-Rohr (1) und dem Mehrkammerrohr (5) verbunden ist, und das Gehäuse (4) mit einem Bluteinlassfenster (13) und einem Blutauslassfenster (14) versehen ist.

6. Katheterpumpe nach Anspruch 5, wobei das Mehrkammerrohr (5) ferner mit einer Ausflusskammer (24) versehen ist, sich die Perfusionskammer (23) von einem Ende des Mehrkammerrohrs (5) zu einem anderen Ende des Mehrkammerrohrs (5) erstreckt, sich die Ausflusskammer (24) von einem Ende des Mehrkammerrohrs (5) bis zu einem anderen Ende des Mehrkammerrohrs (5) erstreckt, ein proximales Ende der Laufrad-Drehwelle (7) mit einem distalen Ende der Übertragungswelle (12) verbunden ist, ein Flüssigkeitskanal (11) in der Laufrad-Drehwelle (7) angeordnet ist, die Laufrad-Drehwelle (7) mit einem Einlass (25) versehen ist, der mit dem Flüssigkeitskanal (11) in Verbindung steht; der Einlass (25) jeweils mit der Perfusionskammer (23), dem Flüssigkeitskanal (11) und der Ausflusskammer (24) in Verbindung steht; Perfusionsflüssigkeit aus der Perfusionskammer (23) fließt, vom proximalen Ende der Laufrad-Drehwelle (7) durch den Einlass (25) in den Flüssigkeitskanal (11) strömt, entlang des Flüssigkeitskanals (11) zum distalen Ende der Laufrad-Drehwelle (7) fließt, aus dem Spalt zwischen der Laufrad-Drehwelle (7) und der Drehvorrichtung (15) austritt, und dann in den menschlichen Körper gelangt, und die Perfusionsflüssigkeit aus der Ausflusskammer (24) austritt.

7. Katheterpumpe nach Anspruch 6, wobei das Mehrkammerrohr (5) ein Innenrohr (26) und ein Außenrohr (27) umfasst, das Innenrohr (26) koaxial an einer Innenseite des Außenrohrs (27) angeordnet ist, die Perfusionskammer (23) zwischen dem Innenrohr (26) und dem Außenrohr (27) angeordnet ist, die Übertragungswelle (12) sich im Innenrohr (26) befindet und die Ausflusskammer (24) zwischen der Übertragungswelle (12) und dem Innenrohr (26) ausgebildet ist.

8. Katheterpumpe nach Anspruch 7, wobei zwischen der Übertragungswelle (12) und dem Innenrohr (26) ein Federrohr (28) angeordnet ist.

9. Katheterpumpe nach Anspruch 6, wobei das proximale Ende der Laufrad-Drehwelle (7) mittels eines Schaltblocks (29) mit dem distalen Ende der Übertragungswelle (12) verbunden ist, der Schaltblock (29) mit einem ersten Kanal (30) versehen ist und der erste Kanal (30) jeweils mit dem Einlass (25), der Perfusionskammer (23) und der Ausflusskammer (24) in Verbindung steht.

10. Katheterpumpe nach Anspruch 9, wobei das proximale Ende der Laufrad-Drehwelle (7) mit Vorsprüngen (31) versehen ist, der Einlass (25) zwischen benachbarten Vorsprüngen (31) ausgebildet ist, der Umschaltblock (29) mit einer Gleitbahn (32) versehen ist, der Vorsprung (31) in Gleitverbindung mit der Gleitbahn (32) steht, ein zweiter Kanal (33) zwischen einer Innenfläche des Vorsprungs (31) und einer Fläche der Gleitbahn (32) ausgebildet ist und der zweite Kanal (33) jeweils mit der Perfusionskammer (23) und dem Flüssigkeitswegkanal (11) in Verbindung steht.

11. Katheterpumpe nach Anspruch 10, wobei der zweite Kanal (33) ferner mit dem ersten Kanal (30) und der Ausflusskammer (24) in Verbindung steht.

12. Katheterpumpe nach Anspruch 9, wobei an einer Außenseite einer Verbindungsstelle zwischen dem proximalen Ende der Laufrad-Drehwelle (7) und dem Schaltblock (29) eine Hüllenabdeckung (34) angeordnet ist, wobei die Hüllenabdeckung (34) jeweils mit der ausdehnbaren Klammer (3) und dem Mehrkammerrohr (5) verbunden ist, in der Hüllenabdeckung (34) eine Öffnung (35) ausgebildet ist und ein distales Ende der Perfusionskammer (23) mittels der Öffnung (35) mit einem distalen Ende der Ausflusskammer (24) in Verbindung steht.

13. Katheterpumpe nach Anspruch 12, wobei die Hüllenabdeckung (34) mit einer Hüllenabdeckungsbegrenzungsstufe (36) versehen ist, wobei die Hüllenabdeckungsbegrenzungsstufe (36) mit dem proximalen Ende der Laufrad-Drehwelle (7) und dem proximalen Ende des Schaltblocks (29) in Eingriff steht, um die Laufrad-Drehwelle (7) axial zu begrenzen und eine Bewegung der Laufrad-Drehwelle (7) in Richtung des proximalen Endes der Katheterpumpe zu verhindern.

14. Katheterpumpe nach Anspruch 12, wobei ein dritter Kanal (37) zwischen dem proximalen Ende der Laufrad-Drehwelle (7) und der Hüllenabdeckung (34) angeordnet ist, ein proximales Ende des dritten Kanals (37) mit der Perfusionskammer (23) in Verbindung steht und das distale Ende des dritten Kanals (37) mit dem menschlichen Körper in Verbindung steht.

15. Katheterpumpe nach Anspruch 14, wobei der dritte Kanal (37) ferner mit dem ersten Kanal (30) und der Ausflusskammer (24) in Verbindung steht.

16. Katheterpumpe nach Anspruch 1, umfassend ein Griffmodul (6), wobei das Griffmodul (6) eine Antriebsdichtungsstruktur umfasst, wobei die Antriebsdichtungsstruktur eine äußere Magnetkomponente (41), eine innere Magnetkomponente (42), eine angetriebene Welle (43) und eine Dichtungsabdeckung (44) umfasst, wobei die innere Magnetkomponente (42) auf der angetriebenen Welle (43) angeordnet ist, die angetriebene Welle (43) mit einer Übertragungswelle (12) mit der Laufrad-Drehwelle (7) verbunden ist, die äußere Magnetkomponente (41) an einer Außenseite der inneren Magnetkomponente (42) angeordnet ist, die äußere Magnetkomponente (41) mit einer mechanischen Kraftquelle verbunden ist, die Dichtungsabdeckung (44) so ausgebildet ist, dass sie die innere Magnetkomponente (42) von der äußeren Magnetkomponente (41) isoliert; und die angetriebene Welle (43) sich relativ zur Dichtungsabdeckung (44) dreht und sich in axialer Richtung bewegt.

17. Katheterpumpe nach Anspruch 16, ferner umfassend ein Pigtail-Rohr (1), eine ausdehnbare Klammer (3), ein Gehäuse (4) und ein Flüssigkeitswegmodul, wobei die Laufrad-Drehwelle (7) mittels einer Drehvorrichtung (15) drehbar mit dem Pigtail-Rohr (1) verbunden ist, das Flüssigkeitswegmodul ein Mehrkammerrohr (5) umfasst, die Übertragungswelle (12) sich in dem Mehrkammerrohr (5) befindet, sich die ausdehnbare Klammer (3) an einer Außenseite der Laufradstruktur (2) befindet, ein distales Ende der ausdehnbaren Klammer (3) mit dem Pigtail-Rohr (1) verbunden ist, ein proximales Ende der ausdehnbaren Klammer (3) mit dem Mehrkammerrohr (5) verbunden ist, das Gehäuse (4) sich an einer Außenseite der ausdehnbaren Klammer (3) befindet, das Gehäuse (4) jeweils mit dem Pigtail-Rohr (1) und dem Mehrkammerrohr (5) verbunden ist, und das Gehäuse (4) mit einem Bluteinlassfenster (13) und einem Blutauslassfenster (14) versehen ist.

18. Katheterpumpe nach Anspruch 17, wobei das Griffmodul (6) ferner einen Perfusionskammerkörper (38) umfasst, wobei eine Perfusionskammer (23) des Mehrkammerrohrs (5) mit einem Perfusionsanschluss des Perfusionskammerkörpers (38) verbunden ist, eine Ausflusskammer (24) des Mehrkammerrohrs (5) mit einem Ausflussanschluss des Perfusionskammerkörpers (38) in Verbindung steht, ein proximales Ende des Mehrkammerrohrs (5) mit der Perfusionskammerkörper (38) verbunden ist, ein proximales Ende der Perfusionskammer (23) nicht mit einem proximalen Ende der Ausflusskammer (24) in Verbindung steht, und die Dichtungsabdeckung (44) mit dem Perfusionskammerkörper (38) verbunden ist.

19. Katheterpumpe nach Anspruch 18, wobei die innere Magnetkomponente (42) auf die angetriebene Welle (43) aufgeschoben und fest mit der angetriebenen Welle (43) verbunden ist, die Dichtungsabdeckung (44) an einer Außenseite der inneren Magnetkomponente (42) angeordnet ist, die äußere Magnetkomponente (41) an einer Außenseite der Dichtungsabdeckung (44) angeordnet ist, ein Öffnungsende (47) der Dichtungsabdeckung (44) hermetisch mit der Perfusionskammerkörper (38) verbunden ist und sowohl die angetriebene Welle (43) als auch die innere Magnetkomponente (42) in axialer Richtung der Dichtungsabdeckung (44) beweglich sind.

20. Katheterpumpe nach Anspruch 19, wobei ein Ende der angetriebenen Welle (43) aus dem Öffnungsende (47) der Dichtungsabdeckung (44) herausragt, zwischen der angetriebenen Welle (43) und dem Perfusionskammerkörper (38) ein Spalt gebildet ist, der Spalt zwischen der angetriebenen Welle (43) und dem Perfusionskammerkörper (38) mit der Ausflusskammer (24) in Verbindung steht, ein weiteres Ende der angetriebenen Welle (43) sich in der Nähe eines Dichtungsendes (48) der Dichtungsabdeckung (44) befindet, ein proximales Ende der Laufrad-Drehwelle (7) fest mit einem distalen Ende der Übertragungswelle (12) verbunden ist, wobei die Bewegung der angetriebenen Welle (43) vom Dichtungsende (48) begrenzt ist und Spalte zwischen der angetriebenen Welle (43) und der Dichtungsabdeckung (44) sowie zwischen der inneren Magnetkomponente (42) und der Dichtungsabdeckung (44) gebildet werden.

21. Katheterpumpe nach Anspruch 16, wobei die angetriebene Welle (43) eine massive Struktur aufweist und ein Ende der angetriebenen Welle (43) mit der Übertragungswelle (12) verbunden ist.

22. Katheterpumpe nach Anspruch 16, wobei die angetriebene Welle (43) eine hohle Struktur aufweist und sich die Übertragungswelle (12) in die angetriebene Welle (43) hinein erstreckt.

23. Katheterpumpe nach Anspruch 18, wobei die Übertragungswelle (12) eine Hohlwelle ist, im Inneren der Übertragungswelle (12) ein Rücklaufkanal angeordnet ist und der Rücklaufkanal mit der Ausflusskammer (24) in Verbindung steht.

24. Katheterpumpe nach Anspruch 16, wobei die angetriebene Welle (43) eine optische Achse ist.

25. Katheterpumpe nach Anspruch 16, wobei die angetriebene Welle (43) einen ersten Wellenteil (50) und einen zweiten Wellenteil (51) umfasst, der erste Wellenteil (50) sich innerhalb der Dichtungsabdeckung (44) befindet, der zweite Wellenabschnitt (51) außerhalb der Dichtungsabdeckung (44) angeordnet ist, an einer Außenseite des zweiten Wellenabschnitts (51) eine Spiralstruktur (52) angeordnet ist und die Drehrichtung der Spiralstruktur (52) entgegengesetzt zur Drehrichtung der angetriebenen Welle (43) ist.

26. Katheterpumpe nach Anspruch 18, wobei ein verschleißfester Einsatz (53) zwischen der angetriebenen Welle (43) und dem Perfusionskammerkörper (38) angeordnet ist, der verschleißfeste Einsatz (53) fest mit der Perfusionskammerkörper (38) verbunden ist, der verschleißfeste Einsatz (53) relativ zur angetriebenen Welle (43) drehbar ist, die angetriebene Welle (43) mit einem Vorsprung (54) versehen ist und der verschleißfeste Einsatz (53) mit dem Vorsprung (54) in Eingriff steht, um die angetriebene Welle (43) axial zu begrenzen und eine Bewegung der angetriebenen Welle (43) in Richtung eines distalen Endes der Katheterpumpe zu verhindern.

27. Katheterpumpe nach Anspruch 18, wobei das Griffmodul (6) ferner ein erstes Diffusionsspannungsrohr (55), ein Motorgehäuse (56), ein zweites Diffusionsspannungsrohr (57) und ein Kabel (58) umfasst, wobei ein Ende des ersten Diffusionsspannungsrohrs (55) mit dem Mehrkammerrohr (5) verbunden ist, ein anderes Ende des ersten Diffusionsspannungsrohrs (55) mit der Perfusionskammerkörper (38) verbunden ist, das erste Diffusionsspannungsrohr (55) mit einem Perfusionsrohr (59) und einem Ausflussrohr (60) versehen ist, ein Ende des Perfusionsrohrs (59) mit dem Perfusionsanschluss verbunden ist, ein Ende des Ausflussrohrs (60) mit dem Ausflussanschluss verbunden ist, das Motorgehäuse (56) an einer Außenseite eines Antriebsmotors (40) angeordnet ist, das Motorgehäuse (56) lösbar mit der Perfusionskammerkörper (38) verbunden ist, das Motorgehäuse (56) mittels einer Sicherungsmutter (61) mit der Perfusionskammerkörper (38) verbunden ist, ein Ende des zweiten Diffusionsdruckrohrs (57) mit dem Motorgehäuse (56) verbunden ist, ein anderes Ende des zweiten Diffusionsdruckrohrs (57) mit dem Kabel (58) verbunden ist und ein Ende des Kabels (58) mit dem Antriebsmotor (40) verbunden ist.

28. Katheterpumpe nach Anspruch 18, wobei das Mehrkammerrohr (5) ein Innenrohr (26) und ein Außenrohr (27) umfasst, das Innenrohr (26) koaxial an einer Innenseite des Außenrohrs (27) angeordnet ist, die Perfusionskammer (23) und ein Druckmesshohlraum (62) zwischen dem Innenrohr (26) und dem Außenrohr (27) angeordnet sind, der Druckmesshohlraum (62) von einer Trennplatte (63) von der Perfusionskammer (23) getrennt ist, der Druckmesshohlraum (62) nicht mit der Perfusionskammer (23) in Verbindung steht, die Übertragungswelle (12) sich im Innenrohr (26) befindet, der Ausflusskammer (24) zwischen der Übertragungswelle (12) und dem Innenrohr (26) vorgesehen ist, der Druckmessraum (62) mittels einer in einer Seitenwand des Außenrohrs (27) ausgebildeten Druckmessöffnung (39) mit dem menschlichen Körper in Verbindung steht, ein Erfassungsraum (64) im Perfusionskammerkörper (38) angeordnet ist, der Erfassungsraum (64) jeweils mit dem Druckmessraum (62) und einem Druckmessrohr (65) in Verbindung steht, das so ausgebildet ist, dass Druckmessflüssigkeit hindurchfließen kann, ein Druckmesselement (66) im Erfassungsraum (64) angeordnet ist und das Druckmesselement (66) so ausgebildet ist, dass es einen durch die Druckmessflüssigkeit übertragenen Innendruck erfasst.

29. Katheterpumpe nach Anspruch 1, wobei in der Laufrad-Drehwelle (7) ein Flüssigkeitskanal (11) angeordnet ist, wobei das Verbindungsteil das Durchgangsloch (18) ist; der Flüssigkeitskanal (11) mit der Durchgangsbohrung (18) in Verbindung steht und die Nabe (9) die Durchgangsbohrung (18) durchquert und an einer Innenwand der Laufrad-Drehwelle (7) eine Filmschicht (19) bildet.

30. Katheterpumpe nach Anspruch 1, wobei der flexible Abschnitt (21) eine Hypotube-Struktur aufweist.

## Revendications

1. Pompe de cathéter comprenant un module fonctionnel, dans laquelle le module fonctionnel comprend une structure de roue (2), la structure de roue (2) comprend un arbre rotatif de roue (7) et une roue (8), la roue (8) comprend un moyeu (9) et plusieurs pales (10), les pales (10) sont agencées sur le moyeu (9), une paroi latérale de l'arbre rotatif de roue (7) est pourvue d'une partie de raccordement (17, 18), le moyeu (9) est relié à la partie de raccordement (17, 18) par moulage par injection monobloc, et la partie de raccordement (17, 18) est configurée pour limiter un mouvement relatif de la roue (8) ;
dans laquelle la partie de raccordement est une rainure (17) et/ou un trou traversant (18) ;
**caractérisée en ce que**
l'arbre rotatif de roue (7) comprend une première section rigide (20), une section flexible (21) et une deuxième section rigide (22) qui sont agencées séquentiellement, la partie de raccordement (17, 18) est située dans la section flexible (21), et la section flexible (21) est apte à être courbée.

2. Pompe de cathéter selon la revendication 1, comprenant en outre un tube en queue de cochon (1), un support expansible (3), un boîtier (4), un tube multi-lumière (5) et un module de poignée (6), dans laquelle l'arbre rotatif de roue (7) est relié de manière rotative au tube en queue de cochon (1), l'arbre rotatif de roue (7) est relié à une source de puissance mécanique dans le module de poignée (6) par l'intermédiaire d'un arbre de transmission (12) dans le tube multi-lumière (5), le support expansible (3) est situé à l'extérieur de la structure de roue (2), une extrémité distale du support expansible (3) est reliée au tube en queue de cochon (1), une extrémité proximale du support expansible (3) est reliée au tube multi-lumière (5), le boîtier (4) est situé à l'extérieur du support expansible (3), le boîtier (4) est relié respectivement au tube en queue de cochon (1) et au tube multi-lumière (5), et le boîtier (4) est pourvu d'une fenêtre d'entrée de sang (13) et d'une fenêtre de sortie de sang (14).

3. Pompe de cathéter selon la revendication 2, dans laquelle une extrémité distale de l'arbre rotatif de roue (7) est pourvue d'un épaulement d'arbre rotatif (16), l'arbre rotatif de roue (7) est relié de manière rotative au tube en queue de cochon (1) par l'intermédiaire d'une structure rotative (15), et une extrémité proximale de la structure rotative (15) coopère avec l'épaulement d'arbre rotatif (16) pour limiter axialement l'arbre rotatif de roue (7) et restreindre le déplacement de l'arbre rotatif de roue (7) vers une extrémité distale de la pompe de cathéter.

4. Pompe de cathéter selon la revendication 1, comprenant un module de circuit fluidique, une structure rotative (15) est prévue entre une extrémité distale de l'arbre rotatif de roue (7) et un tube en queue de cochon (1) ; le module de circuit fluidique comprend un tube multi-lumière (5), le tube multi-lumière (5) comprend une cavité de perfusion (23) ; un liquide de perfusion s'écoule dans l'extrémité distale de l'arbre rotatif de roue (7) depuis la cavité de perfusion (23), sort par un espace entre l'arbre rotatif de roue (7) et la structure rotative (15), puis pénètre dans un corps humain.

5. Pompe de cathéter selon la revendication 4, comprenant en outre un support expansible (3), un boîtier (4) et un module de poignée (6), dans laquelle l'arbre rotatif de roue (7) est relié à une source de puissance mécanique dans le module de poignée (6) par l'intermédiaire d'un arbre de transmission (12) dans le tube multi-lumière (5), le support expansible (3) est situé à l'extérieur de la structure de roue (2), une extrémité distale du support expansible (3) est reliée au tube en queue de cochon (1), une extrémité proximale du support expansible (3) est reliée au tube multi-lumière (5), le boîtier (4) est situé à l'extérieur du support expansible (3), le boîtier (4) est relié respectivement au tube en queue de cochon (1) et au tube multi-lumière (5), et le boîtier (4) est pourvu d'une fenêtre d'entrée de sang (13) et d'une fenêtre de sortie de sang (14).

6. Pompe de cathéter selon la revendication 5, dans laquelle le tube multi-lumière (5) est en outre pourvu d'une cavité d'écoulement sortant (24), la cavité de perfusion (23) s'étend d'une extrémité du tube multi-lumière (5) à une autre extrémité du tube multi-lumière (5), la cavité d'écoulement sortant (24) s'étend d'une extrémité du tube multi-lumière (5) à une autre extrémité du tube multi-lumière (5), une extrémité proximale de l'arbre rotatif de roue (7) est reliée à une extrémité distale de l'arbre de transmission (12), un canal de circuit fluidique (11) est agencé dans l'arbre rotatif de roue (7), l'arbre rotatif de roue (7) est pourvu d'une entrée (25) communiquant avec le canal de circuit fluidique (11), l'entrée (25) communique respectivement avec la cavité de perfusion (23), le canal de circuit fluidique (11) et la cavité d'écoulement sortant (24) ; un liquide de perfusion s'écoule depuis la cavité de perfusion (23), pénètre dans le canal de circuit fluidique (11) depuis l'extrémité proximale de l'arbre rotatif de roue (7) par l'entrée (25), s'écoule le long du canal de circuit fluidique (11) jusqu'à l'extrémité distale de l'arbre rotatif de roue (7), sort par l'espace entre l'arbre rotatif de roue (7) et la structure rotative (15), puis pénètre dans le corps humain, et le liquide de perfusion sort par la cavité d'écoulement sortant (24).

7. Pompe de cathéter selon la revendication 6, dans laquelle le tube multi-lumière (5) comprend un tube intérieur (26) et un tube extérieur (27), le tube intérieur (26) est agencé coaxialement à l'intérieur du tube extérieur (27), la cavité de perfusion (23) est agencée entre le tube intérieur (26) et le tube extérieur (27), l'arbre de transmission (12) est situé dans le tube intérieur (26), et la cavité d'écoulement sortant (24) est formée entre l'arbre de transmission (12) et le tube intérieur (26).

8. Pompe de cathéter selon la revendication 7, dans laquelle un tube à ressort (28) est agencé entre l'arbre de transmission (12) et le tube intérieur (26).

9. Pompe de cathéter selon la revendication 6, dans laquelle l'extrémité proximale de l'arbre rotatif de roue (7) est reliée à l'extrémité distale de l'arbre de transmission (12) par l'intermédiaire d'un bloc de commutation (29), le bloc de commutation (29) est pourvu d'un premier canal (30), et le premier canal (30) communique respectivement avec l'entrée (25), la cavité de perfusion (23) et la cavité d'écoulement sortant (24).

10. Pompe de cathéter selon la revendication 9, dans laquelle l'extrémité proximale de l'arbre rotatif de roue (7) est pourvue de saillies (31), l'entrée (25) est formée entre les saillies adjacentes (31), le bloc de commutation (29) est pourvu d'une glissière (32), la saillie (31) est reliée de manière coulissante à la glissière (32), un deuxième canal (33) est formé entre une surface intérieure de la saillie (31) et une surface de la glissière (32), et le deuxième canal (33) communique respectivement avec la cavité de perfusion (23) et le canal de circuit fluidique (11).

11. Pompe de cathéter selon la revendication 10, dans laquelle le deuxième canal (33) communique en outre avec le premier canal (30) et la cavité d'écoulement sortant (24).

12. Pompe de cathéter selon la revendication 9, dans laquelle un capot de gaine (34) est agencé à l'extérieur d'une jonction entre l'extrémité proximale de l'arbre rotatif de roue (7) et le bloc de commutation (29), le capot de gaine (34) est relié respectivement au support expansible (3) et au tube multi-lumière (5), une ouverture (35) est ménagée dans le capot de gaine (34), et une extrémité distale de la cavité de perfusion (23) communique avec une extrémité distale de la cavité d'écoulement sortant (24) par l'ouverture (35).

13. Pompe de cathéter selon la revendication 12, dans laquelle le capot de gaine (34) est pourvu d'un épaulement de limitation de capot de gaine (36), l'épaulement de limitation de capot de gaine (36) coopère avec l'extrémité proximale de l'arbre rotatif de roue (7) et l'extrémité proximale du bloc de commutation (29) pour limiter axialement l'arbre rotatif de roue (7) et restreindre le déplacement de l'arbre rotatif de roue (7) vers l'extrémité proximale de la pompe de cathéter.

14. Pompe de cathéter selon la revendication 12, dans laquelle un troisième canal (37) est agencé entre l'extrémité proximale de l'arbre rotatif de roue (7) et le capot de gaine (34), une extrémité proximale du troisième canal (37) communique avec la cavité de perfusion (23), et l'extrémité distale du troisième canal (37) communique avec le corps humain.

15. Pompe de cathéter selon la revendication 14, dans laquelle le troisième canal (37) communique en outre avec le premier canal (30) et la cavité d'écoulement sortant (24).

16. Pompe de cathéter selon la revendication 1 comprenant un module de poignée (6), dans laquelle le module de poignée (6) comprend une structure d'étanchéité de transmission, la structure d'étanchéité de transmission comprend un composant magnétique externe (41), un composant magnétique interne (42), un arbre entraîné (43) et un capot d'étanchéité (44), le composant magnétique interne (42) est agencé sur l'arbre entraîné (43), l'arbre entraîné (43) est relié à l'arbre rotatif de roue (7) par l'intermédiaire d'un arbre de transmission (12), le composant magnétique externe (41) est agencé à l'extérieur du composant magnétique interne (42), le composant magnétique externe (41) est relié à une source de puissance mécanique, le capot d'étanchéité (44) est configuré pour isoler le composant magnétique interne (42) du composant magnétique externe (41) ; et l'arbre entraîné (43) tourne par rapport au capot d'étanchéité (44) et se déplace dans une direction axiale.

17. Pompe de cathéter selon la revendication 16, comprenant en outre un tube en queue de cochon (1), un support expansible (3), un boîtier (4) et un module de circuit fluidique, dans laquelle l'arbre rotatif de roue (7) est relié de manière rotative au tube en queue de cochon (1) par l'intermédiaire d'une structure rotative (15), le module de circuit fluidique comprend un tube multi-lumière (5), l'arbre de transmission (12) est situé dans le tube multi-lumière (5), le support expansible (3) est situé à l'extérieur de la structure de roue (2), une extrémité distale du support expansible (3) est reliée au tube en queue de cochon (1), une extrémité proximale du support expansible (3) est reliée au tube multi-lumière (5), le boîtier (4) est situé à l'extérieur du support expansible (3), le boîtier (4) est relié respectivement au tube en queue de cochon (1) et au tube multi-lumière (5), et le boîtier (4) est pourvu d'une fenêtre d'entrée de sang (13) et d'une fenêtre de sortie de sang (14).

18. Pompe de cathéter selon la revendication 17, dans laquelle le module de poignée (6) comprend en outre un corps de cavité de perfusion (38), une cavité de perfusion (23) du tube multi-lumière (5) communique avec un orifice de perfusion du corps de cavité de perfusion (38), une cavité d'écoulement sortant (24) du tube multi-lumière (5) communique avec un orifice d'écoulement sortant du corps de cavité de perfusion (38), une extrémité proximale du tube multi-lumière (5) est reliée au corps de cavité de perfusion (38), une extrémité proximale de la cavité de perfusion (23) ne communique pas avec une extrémité proximale de la cavité d'écoulement sortant (24), et le capot d'étanchéité (44) est relié au corps de cavité de perfusion (38).

19. Pompe de cathéter selon la revendication 18, dans laquelle le composant magnétique interne (42) est emmanché sur l'arbre entraîné (43) et est relié fixement à l'arbre entraîné (43), le capot d'étanchéité (44) est agencé à l'extérieur du composant magnétique interne (42), le composant magnétique externe (41) est agencé à l'extérieur du capot d'étanchéité (44), une extrémité ouverte (47) du capot d'étanchéité (44) est reliée de manière hermétique au corps de cavité de perfusion (38), et l'arbre entraîné (43) et le composant magnétique interne (42) sont tous deux aptes à se déplacer dans une direction axiale du capot d'étanchéité (44).

20. Pompe de cathéter selon la revendication 19, dans laquelle une extrémité de l'arbre entraîné (43) fait saillie depuis l'extrémité ouverte (47) du capot d'étanchéité (44), un espace est formé entre l'arbre entraîné (43) et le corps de cavité de perfusion (38), l'espace entre l'arbre entraîné (43) et le corps de cavité de perfusion (38) communique avec la cavité d'écoulement sortant (24), une autre extrémité de l'arbre entraîné (43) est proche d'une extrémité d'étanchéité (48) du capot d'étanchéité (44), une extrémité proximale de l'arbre rotatif de roue (7) est reliée fixement à une extrémité distale de l'arbre de transmission (12), le mouvement de l'arbre entraîné (43) est limité par l'extrémité d'étanchéité (48), et des espaces sont formés respectivement entre l'arbre entraîné (43) et le capot d'étanchéité (44), et entre le composant magnétique interne (42) et le capot d'étanchéité (44).

21. Pompe de cathéter selon la revendication 16, dans laquelle l'arbre entraîné (43) présente une structure pleine, et une extrémité de l'arbre entraîné (43) est reliée à l'arbre de transmission (12).

22. Pompe de cathéter selon la revendication 16, dans laquelle l'arbre entraîné (43) présente une structure creuse, et l'arbre de transmission (12) s'étend dans l'arbre entraîné (43).

23. Pompe de cathéter selon la revendication 18, dans laquelle l'arbre de transmission (12) est un arbre creux, un canal de retour est agencé à l'intérieur de l'arbre de transmission (12), et le canal de retour communique avec la cavité d'écoulement sortant (24).

24. Pompe de cathéter selon la revendication 16, dans laquelle l'arbre entraîné (43) est un axe optique.

25. Pompe de cathéter selon la revendication 16, dans laquelle l'arbre entraîné (43) comprend une première partie d'arbre (50) et une deuxième partie d'arbre (51), la première partie d'arbre (50) est située à l'intérieur du capot d'étanchéité (44), la deuxième partie d'arbre (51) est située à l'extérieur du capot d'étanchéité (44), une structure hélicoïdale (52) est agencée à l'extérieur de la deuxième partie d'arbre (51), et un sens de rotation de la structure hélicoïdale (52) est opposé à un sens de rotation de l'arbre entraîné (43).

26. Pompe de cathéter selon la revendication 18, dans laquelle un insert résistant à l'usure (53) est agencé entre l'arbre entraîné (43) et le corps de cavité de perfusion (38), l'insert résistant à l'usure (53) est relié fixement au corps de cavité de perfusion (38), l'insert résistant à l'usure (53) peut tourner par rapport à l'arbre entraîné (43), l'arbre entraîné (43) est pourvu d'une saillie (54), et l'insert résistant à l'usure (53) coopère avec la saillie (54) pour limiter axialement l'arbre entraîné (43) et restreindre le déplacement de l'arbre entraîné (43) vers une extrémité distale de la pompe de cathéter.

27. Pompe de cathéter selon la revendication 18, dans laquelle le module de poignée (6) comprend en outre un premier tube de diffusion de contrainte (55), un boîtier de moteur (56), un deuxième tube de diffusion de contrainte (57) et un câble (58), une extrémité du premier tube de diffusion de contrainte (55) est reliée au tube multi-lumière (5), une autre extrémité du premier tube de diffusion de contrainte (55) est reliée au corps de cavité de perfusion (38), le premier tube de diffusion de contrainte (55) est pourvu d'un tube de perfusion (59) et d'un tube d'écoulement sortant (60), une extrémité du tube de perfusion (59) communique avec l'orifice de perfusion, une extrémité du tube d'écoulement sortant (60) communique avec l'orifice d'écoulement sortant, le boîtier de moteur (56) est agencé à l'extérieur d'un moteur d'entraînement (40), le boîtier de moteur (56) est relié de manière amovible au corps de cavité de perfusion (38), le boîtier de moteur (56) est relié au corps de cavité de perfusion (38) par l'intermédiaire d'un écrou de verrouillage (61), une extrémité du deuxième tube de diffusion de contrainte (57) est reliée au boîtier de moteur (56), une autre extrémité du deuxième tube de diffusion de contrainte (57) est reliée au câble (58), et une extrémité du câble (58) est reliée au moteur d'entraînement (40).

28. Pompe de cathéter selon la revendication 18, dans laquelle le tube multi-lumière (5) comprend un tube intérieur (26) et un tube extérieur (27), le tube intérieur (26) est agencé coaxialement à l'intérieur du tube extérieur (27), la cavité de perfusion (23) et une cavité de mesure de pression (62) sont agencées entre le tube intérieur (26) et le tube extérieur (27), la cavité de mesure de pression (62) est séparée de la cavité de perfusion (23) par une plaque de séparation (63), la cavité de mesure de pression (62) ne communique pas avec la cavité de perfusion (23), l'arbre de transmission (12) est situé dans le tube intérieur (26), la cavité d'écoulement sortant (24) est prévue entre l'arbre de transmission (12) et le tube intérieur (26), la cavité de mesure de pression (62) communique avec un corps humain par l'intermédiaire d'un trou de mesure de pression (39) formé dans une paroi latérale du tube extérieur (27), une cavité de détection (64) est agencée dans le corps de cavité de perfusion (38), la cavité de détection (64) communique respectivement avec la cavité de mesure de pression (62) et un tube de mesure de pression (65) qui est configuré pour permettre le passage d'un liquide de mesure de pression, un élément de mesure de pression (66) est agencé dans la cavité de détection (64), et l'élément de mesure de pression (66) est configuré pour détecter une pression interne transmise par le liquide de mesure de pression.

29. Pompe de cathéter selon la revendication 1, dans laquelle un canal de circuit fluidique (11) est agencé dans l'arbre rotatif de roue (7), la partie de raccordement est le trou traversant (18) ; le canal de circuit fluidique (11) communique avec le trou traversant (18), et le moyeu (9) traverse le trou traversant (18) et forme une couche de film (19) sur une paroi intérieure de l'arbre rotatif de roue (7).

30. Pompe de cathéter selon la revendication 1, dans laquelle la section flexible (21) présente une structure d'hypotube.
